# EUROPEAN PATENT SPECIFICATION

(11) **EP 0 510 086 B1**
(45) Date of publication and mention of the grant of the patent: **11.12.1996**
(21) Application number: 91903087.4
(22) Date of filing: 11.01.1991
(51) Int. Cl.: A61K 9/133, A61K 9/127, B01J 13/02

(54) **INTERDIGITATION-FUSION LIPOSOMES AND GELS**
INTERDIGITATION-FUSIONSLIPOSOMEN UND GELE
LIPOSOMES ET GELS A FUSION D'INTERDIGITATION

(30) Priority: 12.01.1990 US 464528
(43) Date of publication of application: 28.10.1992
(73) Proprietor: THE LIPOSOME COMPANY, INC., Princeton, NJ 08540 (US)
(72) Inventor: BONI, Lawrence, T., Monmouth Junction, NJ 08852 (US); JANOFF, Andrew, S., Yardley, PA 19067 (US); MINCHEY, Sharma, R., Monmouth Junction, NJ 08852 (US); PERKINS, Walter, R., Plainsboro, NJ 08536 (US); SWENSON, Christine, E., Princeton Junction, NJ 08550 (US); AHL, Patrick, L., Princeton, NJ 08540 (US)
(74) Representative: Warcoin, Jacques
(86) International application number: US9100259
(87) International publication number: WO9110422

(56) References cited:
- US-A- 4 515 736
- US-A- 4 544 545
- US-A- 4 560 665
- US-A- 4 789 633
- US-A- 4 877 561
- US-A- 4 921 706
- US-A- 4 944 948
- US-A- 4 962 022
- Biochim Biophys Acts, Volume 818, (1985) D.S. MCCONNELL et al., "Phospholipid Vesicle fusion and drug loading: temperature, solute and cholesterol effects, and a rapid preparation for solute - loaded vesides", see pages 13-22.
- Biohim Biophys Acta, Volume 775 (1984) L.T. BONI et al., "Aggregation and Fusion of Unilamellar vesicles by poly(Ethylene Glycol)", see pages 409-418.
- Progress Lipid Research, Volume 27 (1988) J.L. SLATER et al., "Interdigitated Bilayer Membranes", see pages 325-359.
- Biochemistry, Volume 28, (1989) K.M. EUM et al., "Temperature-Induced Fusion of small unilamellar vesicles formed from saturated long chain lecithins and diheptanoylphospharidyl choline", see pp. 8206-8213

## Description

### Field of the Invention

This invention relates to interdigitation-fusion (IF) liposomes and gels. These liposomes and gels capture high solute to lipid ratios. The term solute encompasses bioactive agents, including bioactive agent. This invention also relates to the discovery of interdigitation of lipids to produce IF gels and liposomes, and the further discovery that such interdigitation to form liposomes according to the present invention is size dependent.

The present invention relates to a method for producing IF liposomes and gels. In the method of the present invention, liposomes formed by sonication, extrusion or alternative size reduction processes such as homogenization to the appropriate size are fused in the presence of a suitable inducer. This process produces a composition of the present invention in gel form. The gel itself can be employed, for example, for delivery of bioactive agents, or can be used to form IF liposomes, which in turn possess very high internal volumes and encapsulate large amounts of solute.

To produce IF liposomes from the gels, the gels are incubated at a temperature usually but not necessarily above the transition temperature (Tm) of the lipid used, such that liposomes are formed. The temperature required by the methods of the invention is that temperature for any given mixture of lipid, solute and inducer that induces a change in the material properties of the mixture thereby producing the IF liposomes of the invention. The liposomes formed from IF gels are IF liposomes. Preferably, but not necessarily, the inducer is also removed during the incubation step. The result is a composition comprising liposomes containing high solute to lipid ratios.

### Background of the Invention

The therapeutic properties of many drugs may be dramatically improved by the administration in a liposomally encapsulated form [See, for example P.M. Shek and R.F. Barber, Mod. Med. Canada, 41, 314-382, (1986)]. In certain cases, for example, in the administration of amphotericin B and doxorubicin [Lopez-Berestein, et al., J. Infect. Dis., 151, 704-710, (1985) and Rahman, et al., Cancer Res., 40, 1532 (1980)] toxicity is reduced while efficacy is maintained or even increased. The benefit obtained from liposomally encapsulated agents may be fortuitous, and likely results from the altered pharmacokinetics and biodistribution of the entrapped drug. [Ostro, et al., Amer. J. Hosp. Pharm., Vol. 46 Aug 1989]

A number of methods are presently available for "charging" liposomes with bioactive agents. For example, in a liposome-drug delivery system, a bioactive agent such as a drug may be entrapped in the liposome and then administered to the patient to be treated. See, for example, Rahman et al., U.S. Patent No. 3,993,754; Sears, U.S. Patent No. 4,145,410; Papahadjopoulos, et al., U.S. Patent No. 4,235,871; Lenk et al., U.S Patent No. 4,522,803; and Fountain et al., U.S. Patent No. 4,588,578. In addition to this basic method for entrapping a bioactive agent, if the bioactive agent is lipophilic, it may associate with the lipid bilayer. However, many of the pharmaceutical formulations produced utilizing the traditional methods suffer from the disadvantage of low drug to lipid ratio, scaleup problems and the use of toxic solvents.

In addition to the above-described methods, numerous bioactive agents have been shown to accumulate in liposomes in response to an imposed proton or ionic gradient known as "remote loading" [See, for example Mayer, et al., Biochim. Biophys. Acta, 857, 123, (1986), Mayer, et al., Biochemistry, 27, 2053, (1988) and M.B. Bally, et al., Chem. Phys. Lipids, 47, 97, (1988)]. This loading technique allows independent variation of any of the liposomal parameters. Much higher drug to lipid ratios can be achieved in comparison to conventional techniques [Mayer, et al. Chem. Phys. Lipids, 40, 333 (1986)]. The procedures and materials for remote loading are disclosed in Bally et al., Application Serial No. 284,751, filed December 12, 1988 and Mayer et al., filed January 4, 1991, [TLC-133B], which is a continuation of Application Serial No. 164,557, filed March 7, 1988, now abandoned.

Liposomes are completely closed lipid bilayer membranes which contain entrapped aqueous volume. Liposomes may be unilamellar (single bilayer membrane) or multilamellar (onion-like structures characterized by multiple bilayer membranes, each separated from the next by an aqueous layer). The bilayer is composed of two lipid monolayers having a hydrophobic "tail" region and a hydrophilic "head" region. In the membrane bilayer, the hydrophobic (nonpolar) "tails" of the lipid monolayers orient toward the center of the bilayer, whereas the hydrophilic (polar) "heads" orient toward the aqueous phase. The basic structure of liposomes may be made by a variety of techniques known in the art.

One class of liposomes that may be used in the practice of the invention are those characterized as having substantially equal lamellar solute distribution. This class of liposomes is denominated is stable plurilamellar vesicles (SPLV) as defined in U.S. Patent No. 4,522,803 to Lenk, et al., monophasic vesicles as described in U.S. Patent No. 4,588,578 to Fountain, et al., and frozen and thawed multilamellar vesicles (FAT MLV) wherein the vesicles are exposed to at least one freeze and thaw cycle; this procedure is described in Billy et al., U.S. Patent No. 4,975,282, issued December 4, 1990, entitled "Multilamellar Liposomes Having Improved Trapping Efficiencies"

Liposomal encapsulation could potentially provide numerous beneficial effects for a wide variety of pharmaceutical agents and a high drug to lipid ratio should prove important in realizing the potential of liposomally encapsulated agents. The use of liposomes to administer drugs has raised problems with regard both to drug encapsulation and drug release during therapy. For example, there is a continuing need to increase drug to lipid ratios so as to minimize the lipid load presented to the patient.

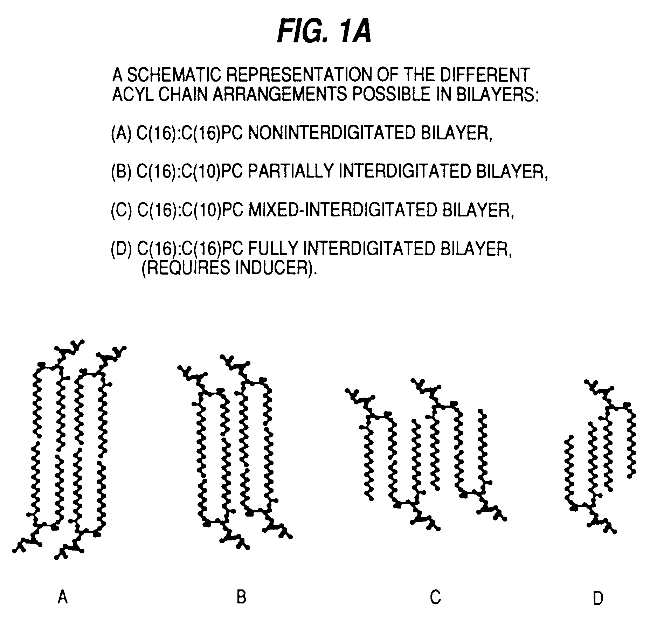

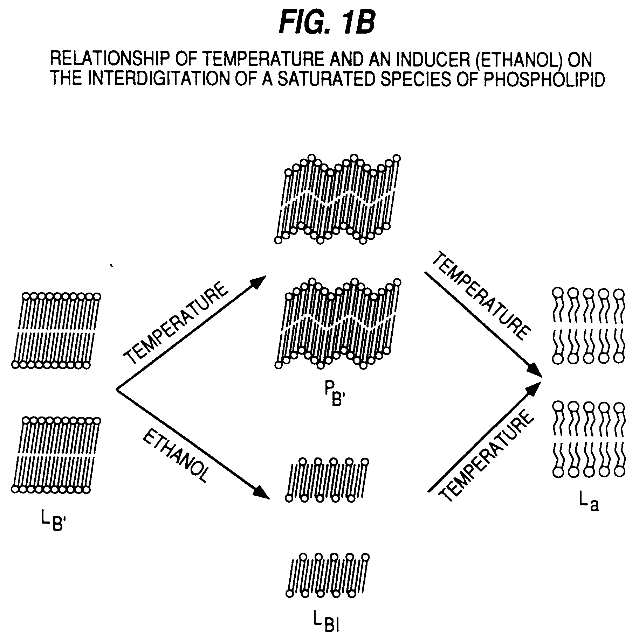

Interdigitation of lipids is a phenomenon which has been recently explored in considerable detail by James L. Slater and Ching-Hsien Huang in Progress Lipid Res., 27, 325-359, 1988. In general, the art describes the interdigitation of various lipid species resulting from either the presence of various inducers and/or acyl chain length asymmetry (See Figures 1A and 1B). There has been no report in the literature, however, of the size dependency for fusing liposomes during interdigitation to produce the IF gels and liposomes of the present invention.

### Objects of the Present Invention

It is an object of the present invention to provide interdigitation-fusion gels and liposomes, which may be used for delivering solute in a number of applications, including therapeutic applications.

It is another object of the present invention to provide an interdigitation-fusion gel which contains saturated lipid and may additionally contain non-saturated lipids and effective concentrations of bioactive agents for formulation into compositions for topical or oral administration to a mammal, including humans.

It is a further object of the present invention to provide a method for producing interdigitation-fusion liposomes and gels which accumulate high concentrations of bioactive agents.

It is yet another object of the present invention to provide pharmaceutical compositions based upon the interdigitition-fusion gels and liposomes of the present invention.

It is in additional object of the present invention to provide therapeutic compositions for treating animals, especially mammals, including humans with interdigitition-fusion liposomes and gels having a high solute to lipid ratio.

It is still another object of the present invention to provide methods for making the interdigitation gels and IF liposomes of the present invention.

It is another object of the present invention to provide a novel method for trapping bioactive agents.

These and other objects of the present invention may be readily understood from the detailed description of the invention which is set forth herein.

### Summary of the Invention

The present invention relates to interdigitation-fusion (IF) liposomes and gels which can contain solute. These liposomes and gels capture high solute to lipid ratios, including bioactive agent. These IF gels and liposomes find use in a number of applications, including cosmetic, pharmaceutical and agricultural applications. In combination with bioactive agents, these liposomes and gels may be administered topically or systemically to plants and animals, especially mammals, including humans. In addition to the above applications, the IF gels and liposomes of the present invention are also useful in combination with resin technology, in particular, paint technology.

The compositions of the present invention comprise a sized liposome, preferably 0.4 microns in diameter or less, more preferably 0.05 microns in diameter or less, and most preferably 0.025 microns in diameter, in combination with a solute, for example a bioactive agent and an amount of an interdigitation inducer effective to fuse the liposomes to produce an IF gel. The initial liposomes may alternatively be FAT MLVs. IF liposomes may be produced from the IF gels of the present invention. Preferably, in the IF liposomes and gels of the present invention, a saturated lipid, for example, dipalmitoylphosphatidylcholine (DPPC),
dimyristoylphosphatidylcholine (DMPC),
di-0-hexadecylphosphatidylcholine and distearoylphosphatidylcholine, as well as lipids in which the unsaturated carbon-carbon double bonds in the acyl side chains of the lipid are in the trans configuration, such as transdielaidoylphosphatidylcholine and dipalmitelaidoyl- phosphatidylcholines, or mixed fatty acid lipids such as palmitoyloleoylphosphatidylcholine (POPC) or 1-stearoyl-2-oleoyl phosphatidylcholine (SOPC), as well as other unsaturated lipids, may also be used.

In certain embodiments, unsaturated lipids can be employed in combination with the saturated lipids of the invention. It is generally preferred that when DOPC is the unsaturated lipid employed, it be used with the saturated lipid DPPC in no more than a proportion of 50 mole percent unsaturated lipid.

The interdigitation-fusion gel that is produced when sized liposomes are fused, preferably in the presence of an inducer, which may or may not contain a bioactive agent, may be used without further modification, or alternatively, the gel may be further modified, for example, by usually but not necessarily heating to a temperature above the lipid transition temperature (Tm) but in any case incubating the mixture to a temperature which will induce a change in the material properties of the mixture and thus induce the formation of IF liposomes from the IF gels of the invention, and further possibly removing the interdigitation inducer contained therein, to produce IF liposomes.

The IF liposomes of the present invention may be used to capture surprisingly high solute to lipid ratios, including bioactive agents. These IF liposomes may be used without further modification or they may be further sized to produce liposomes of varying and/or homogeneous size using techniques and methodologies readily available in the art, and which will be reviewed hereinbelow.

In the present invention, the preferred interdigitation inducer is a short-chain alcohol, such as those having 1 to 4 carbon atoms, preferably ethanol because of the ease with which it can be removed to produce IF liposomes from the IF gels of the present invention. However, any inducer that produces a fused IF gel from sized liposomes may be used in embodiments of the present invention.

Exemplary inducers for use in the present invention include, for example, polyols such as glycerol, ethylene glycol, short-chain alcohols such as methanol, ethanol, propanol, isopropanol and n-butanol and anesthetics such as chlorpromazine, tetracaine, phenylethanol, benzyl alcohol and phenylbutanol, and others including polymixin, myelin basic protein, choline, acetylcholine, Tris buffer and chaotropic salts such as, for example, thiocyanate. Ethanol, however, is preferred because of its ease of removal and pharmaceutical compatibility. The amount of inducer used in the present invention comprises an amount effective for producing interdigitation-fusion gels from sized liposomes. It is to be noted that in certain embodiments of the present invention the saturated lipid used to make IF gels and liposomes of the present invention may be a self-inducer, i.e., the saturated lipid will produce IF gels and liposomes of the present invention without the need to add an inducer. In particular, the use of Di-0-hexadecylphosphatidylcholine (DHPC) in this aspect of the present invention as exemplary, is noted. Alternatively or additionally, as noted herein, a solute which may be a bioactive agent may itself also be an inducer.

The IF liposomes and gels of the present invention may contain concentrations of virtually any solute, including bioactive agents such as vitamins, hormonal agents, antimetabolites, antimicrobial agents, antifungal agents, local anaesthetics, bronchodilators, beta-adrenergic blockers, antihypertensive agents, antidepressants, anticonvulsants, antihistamines, antimalarial agents, analgesics, antibiotics, immunogens, immunomodulators, antigens, nutrients, proteins, peptides, nucleosides, oligo and polynucleotides, ribonucleic acid (RNA) and deoxyribonucleic acid (DNA) and analogs of RNA and DNA, antineoplastic agents, antihistaminic agents, neuropharmacologic agents including sedatives and hypnotics, steroidal and nonsteroidal antiinflammatory agents, diuretic agents, antiarrhythmic agents and vascular dilating agents, among others, including radiographic contrast agents, nuclear magnetic resonance (NMR) contrast agents and antiviral agents. Additional bioactive agents for use in the present invention include nutrients such as proteins, fatty acids and carbohydrates. In certain preferred embodiments of the present invention, radiocontrast agents, NMR contrast agents, peptides and certain antibiotics, for example, cephalosporins are utilized in the present invention. Exemplary radiocontrast agents for use in the present invention include, for example, iohexol, iopamidol, ioxoglate, iotrolan, ioversol, iothalamate, iodimide, iodipamide, iopentol, iodixanol, metrizamide, mixtures thereof and their pharmaceutically acceptable salts. Bioactive agents can be naturally occurring, synthetic, or semi-synthetic antimicrobial agents. Exemplary antimicrobial agents are aminoglycosides such as: gentamicin, amikacin and tobramycin. In preferred compositions of the present invention, the IF gels and/or liposomes contain high concentrations of the bioactive agent. In general, the bioactive agent/lipid weight ratio of the IF gels and liposomes of the present invention are as high as from 1:10 to 15:1. Of course, these weight ratios are exemplary only and in certain cases it may be necessary to provide drug and lipid in weight ratios above or below these ratios.

In certain embodiments of the present invention the solute or bioactive agent can also function as the inducer. In such cases, it is not necessary to remove the inducer/bioactive agent as in other embodiments of the present invention.

Suitable bioactive agents for use in the present invention include any agent which exhibits biological activity when administered topically or systemically in the liposomes or gels of the present invention. Numerous bioactive agents may be included with the IF gels of the present invention, preferably for topical or oral delivery. The same agents may be included in the IF liposomes of the present invention for topical administration.

The present invention also relates to a method for producing IF liposomes and gels containing a bioactive agent of varying concentration. In the method of the present invention, sized liposomes, preferably 0.4 µm in diameter or less, and more preferably 0.05 µm in diameter or less and most preferably 0.025 µm in diameter, formed by sonication, extrusion, homogenization or an alternative process, or alternatively, FAT MLVs, are fused in the presence of ethanol or other suitable inducer. Depending upon the interaction of the bioactive agent with the lipids used in the IF gels and liposomes of the present invention, the agent may be added before or after the inducer is added. The addition of inducer results in the IF gel of the present invention. The gel including bioactive agent can be administered to a patient, or alternatively, converted to IF liposomes of the present invention.

To produce IF liposomes of the present invention, the IF gels are exposed to a temperature usually but not necessarily above the transition temperature of the lipid used and, additionally, the inducer may be removed. The temperature required by the methods of the invention is that temperature, for any given mixture of lipid, solute or inducer that produces a change in the material properties of the mixture thereby forming the IF gels and IF liposomes of the invention. The result is a composition comprising IF liposomes containing high concentrations of bioactive agent.

The IF liposomes produced by this method may vary in size generally from 100 µm to 0.025 µm, more preferably 20 µm to 0.025 µm, and may contain high concentrations of bioactive agent. These IF liposomes may be further size reduced using any of the techniques available in the art.

### Brief Description of the Drawing

Figure 1A is a schematic representation of the different acyl chain arrangements possible in bilayers. A represents a noninterdigitated bilayer comprising a phospholipid containing a symmetrical, saturated phospholipid C(16):C(16)phosphatidylcholine. B represents a partially interdigitated bilayer comprising an asymmetrical saturated phospholipid C(16):C(10)phosphatidylcholine. C represents a mixed interdigitated bilayer comprising C(16):C(10)phosphatidylcholine. D represents a fully interdigitated bilayer comprising C(16):C(16)phosphatidylcholine in combination with an effective amount of an inducer.

Figure 1B is a schematic representation showing the effect of temperature and an inducer (ethanol) on the interdigitation of a saturated species of phospholipid.

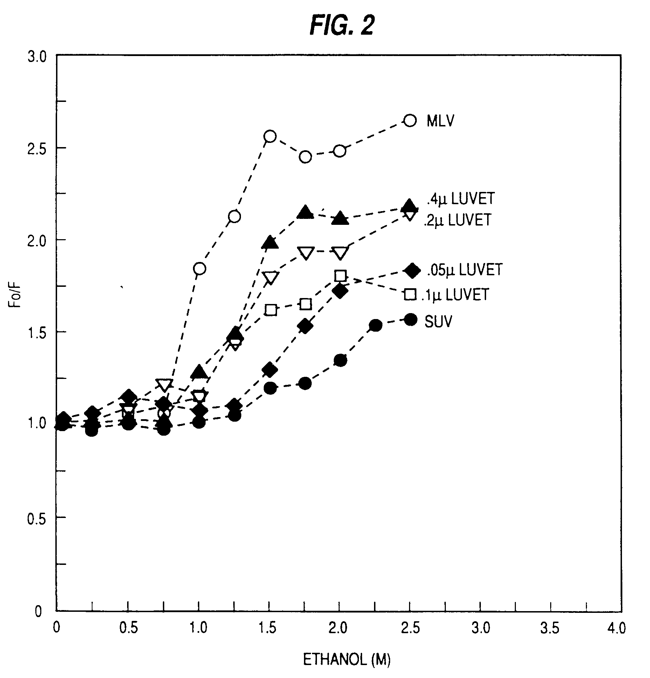

Figure 2 represents interdigitation of dipalmitoylphosphatidylcholine (DPPC) liposomes as a function of the initial size of the liposomes and the concentration of ethanol. Interdigitation is determined by diphenylhexatriene (DPH) fluorescence intensity. DPH fluoresces maximally when incorporated into the lipid bilayer. Interdigitation results in the reorientation of DPH with a concomitant decrease in fluorescence. Fo= DPH fluorescence in the absence of ethanol. F = DPH fluorescence in the presence of ethanol. Excitation = 351 nm. Emission was detected between 380 and 580 nm and quantitated by weighing.

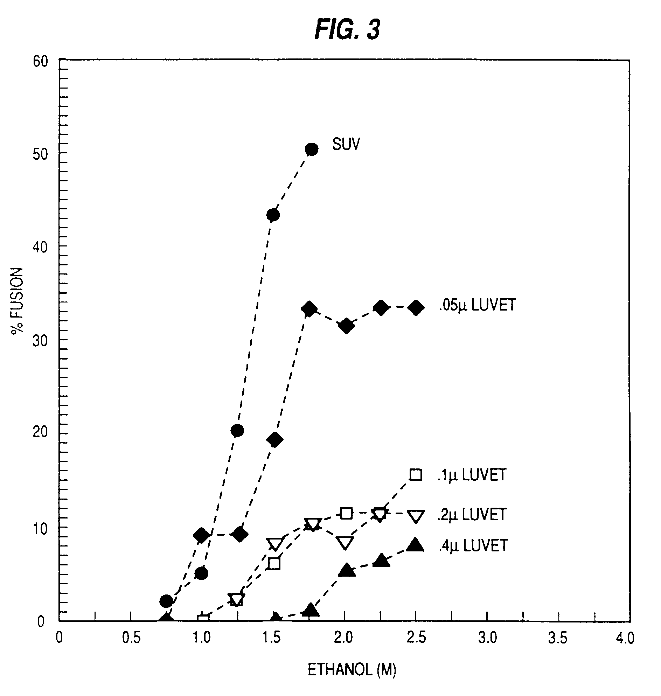

Figure 3 represents lipid mixing of DPPC liposomes as a function of size and ethanol concentration as judged by resonance energy transfer (RET) between NBD-PE and rhodamine-PE incorporated together in a marker population of liposomes.

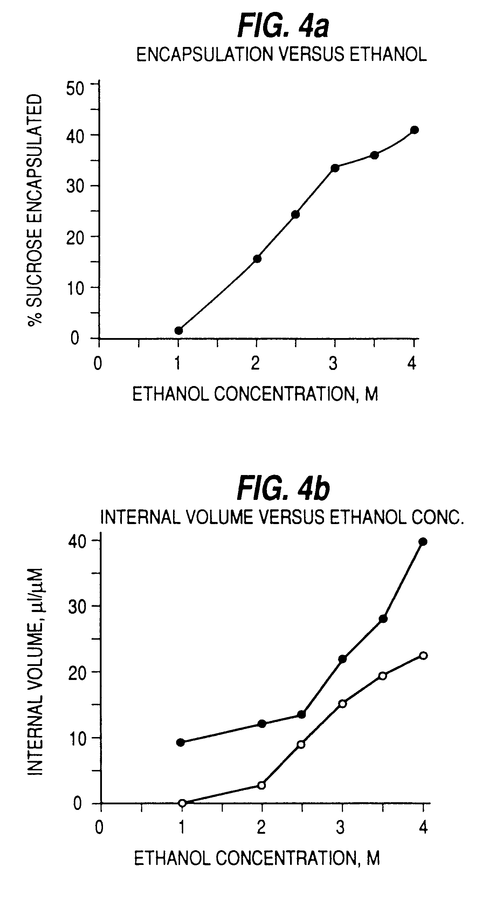

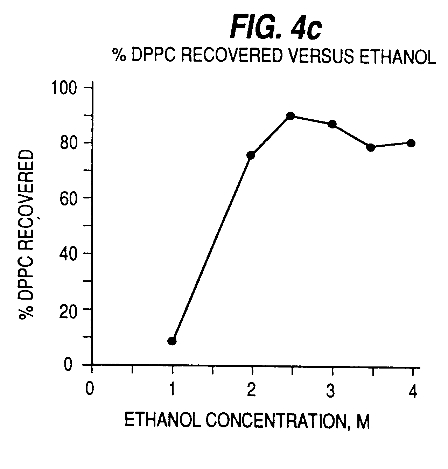

Figure 4 A graphically represents the ¹⁴C sucrose encapsulation percentage as a function of ethanol concentration. The internal volume of DPPC IF liposomes as a function of increased ethanol concentration is shown in Figure 4B, solid circles representing internal volume determined by ¹⁴C sucrose encapsulation; open circles refer to the CAT 1 EPR measurement. Figure 4C represents the percentage of DPPC recovered as a result of failure of IF liposomes to form at 1.0 M ethanol concentrations, with the result being the SUVs remaining did not successfully centrifuge.

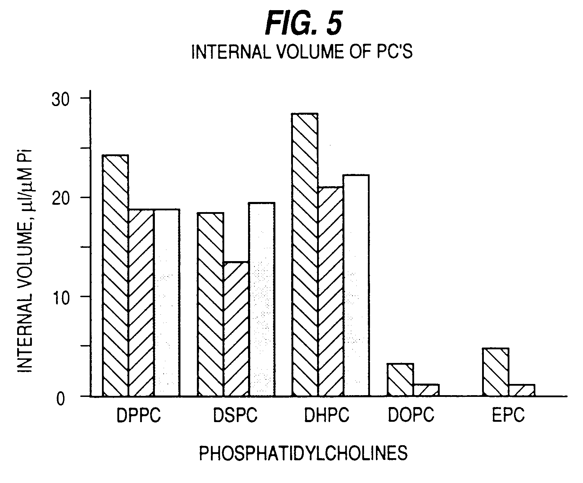

Figure 5 shows internal volumes of IF liposomes formed from various lipids using ¹⁴C sucrose, TEMPONE EPR and CAT 1 EPR methods (solid, diagonal, and shaded bars, respectively).

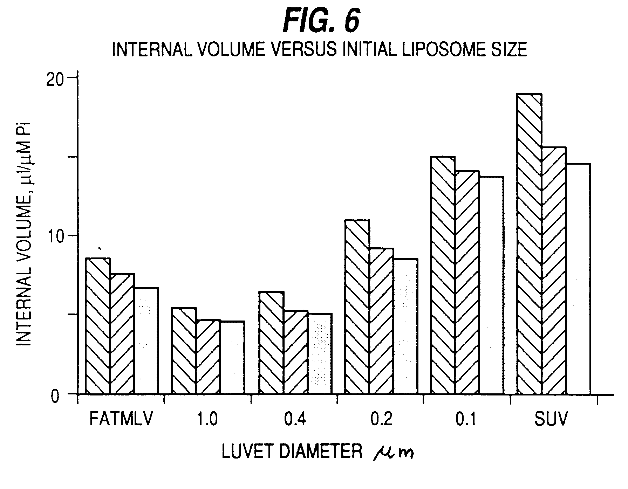

Figure 6 shows the internal volume of DPPC IF liposomes as a function of initial size of liposomes prior to the addition of ethanol. Internal volumes of these liposomes were calculated by ¹⁴C sucrose encapsulation as well as CAT 1 EPR and TEMPONE EPR methods (solid, diagonal and shaded bars, respectively).

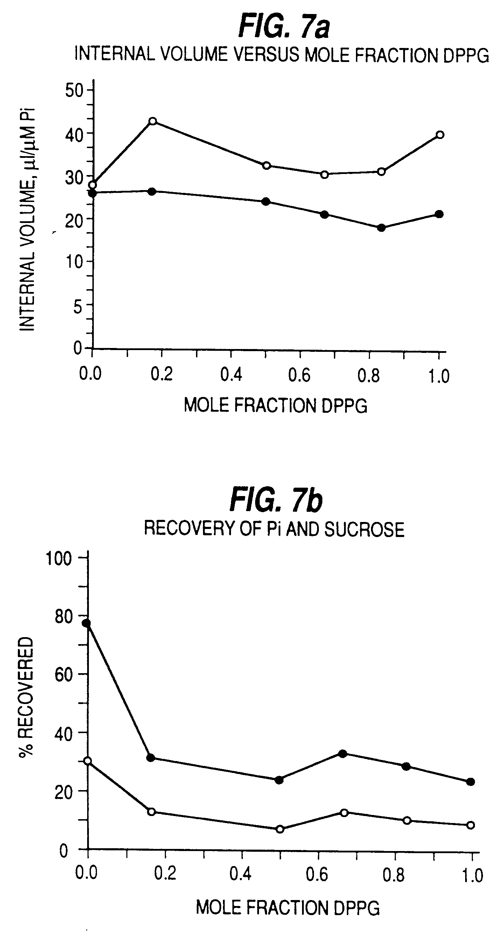

Figure 7 graphically represents the incorporation of DPPG into DPPG-DPPC IF liposomes. The internal volumes of the IF liposomes are shown in Figure 7A as a function of mole fraction of DPPG (volumes measured by ¹⁴C encapsulation, open circles; volumes measured by the broadening agent TEMPONE EPR technique, closed circles). Figure 7 B shows the percent recovery of Pi (closed circles) and ¹⁴C labelled sucrose (open circles) as a function of DPPG.

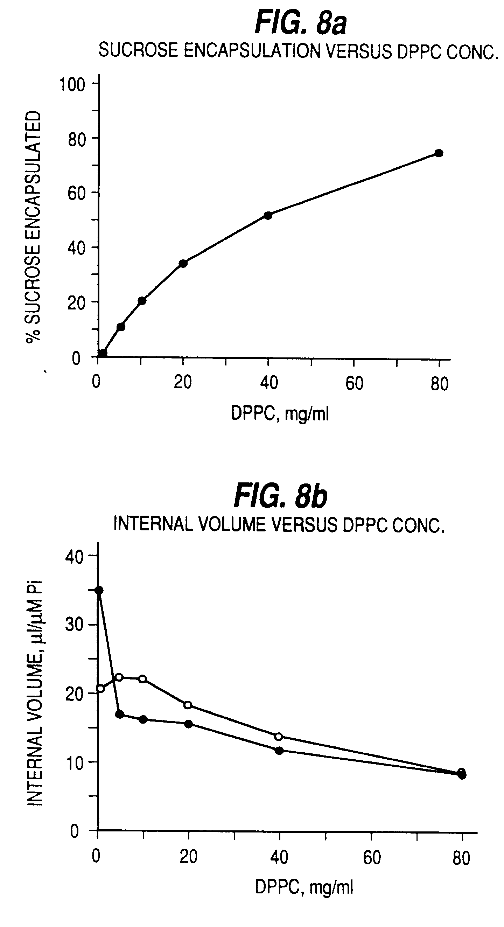

Figure 8 graphically represents the internal volume and encapsulation as a function of initial DPPC concentration, wherein Figure 8 A shows that the encapsulation percentage of sucrose increases with the initial DPPC lipid concentration. Figure 8 B shows the internal volume of the DPPC IF liposomes measured by both the ¹⁴C sucrose method (closed circles) and the EPR method (open circles).

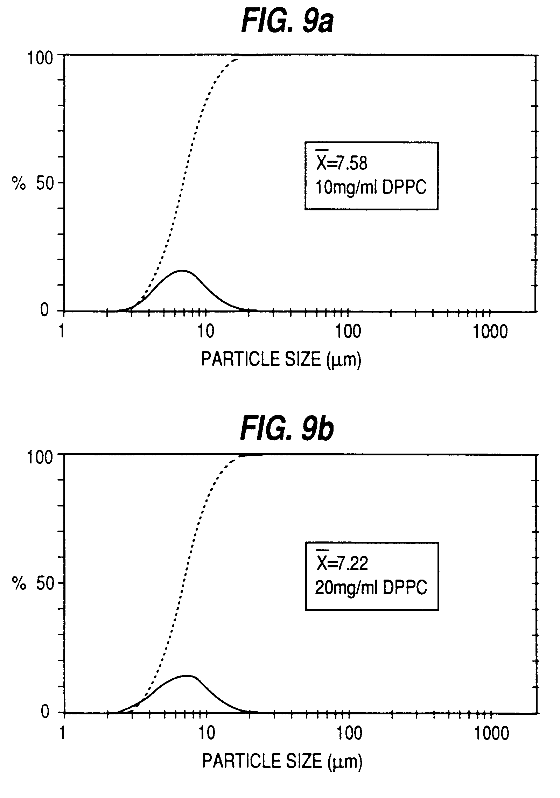

Figure 9 are Malvern particle size distributions of IF liposomes at (A) 10 mg/ml and (B) 20 mg/ml lipid.

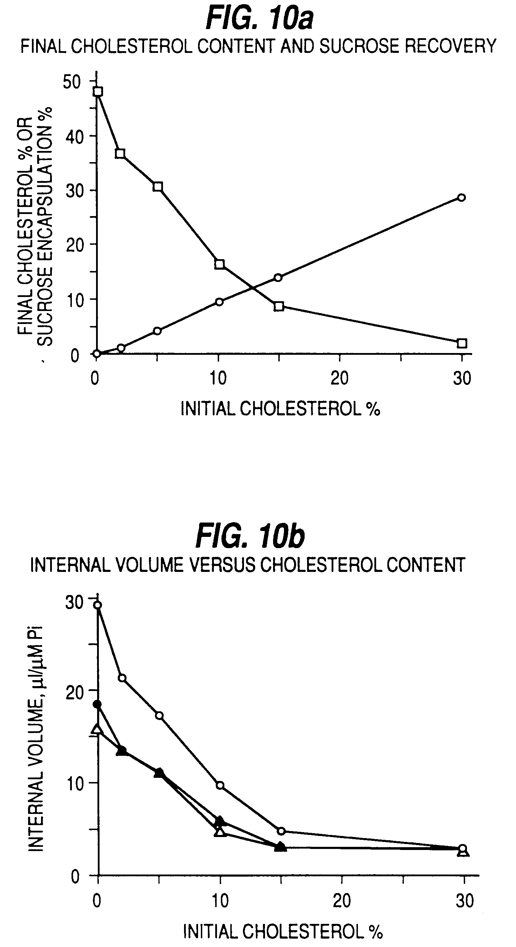

Figure 10 graphically represents the effect of cholesterol on formation of DPPC IF liposomes. Figure 10A show the "final" cholesterol concentration of the IF liposomes (open circles) and the final percentage of ¹⁴C sucrose encapsulated (open squares) as a function of initial cholesterol percent of the liposomes prior to addition of ethanol. Figure 10B shows the decrease in internal volume of the DPPC-cholesterol IF liposomes as a function of cholesterol content (¹⁴C sucrose encapsulation, CAT 1 EPR and TEMPONE EPR methods; open circles, open triangles and closed circles respectively).

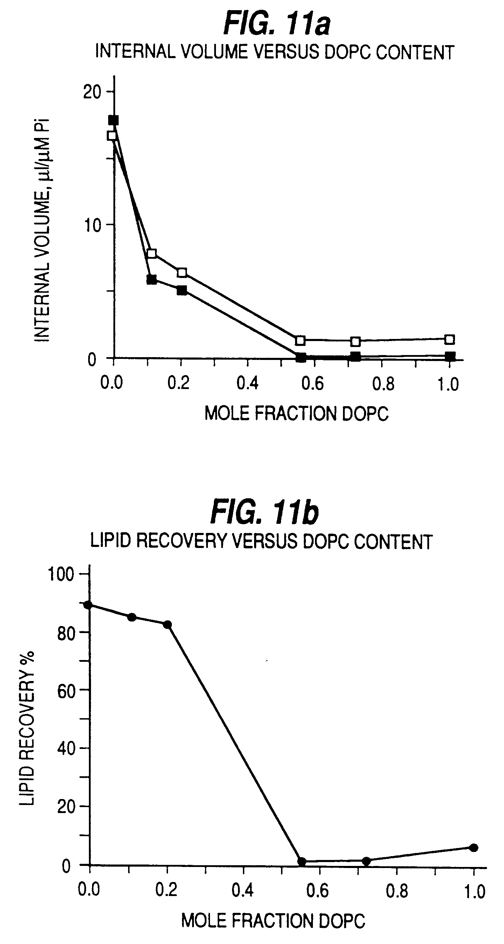

Figure 11 graphically represents the effect of dioleoylphosphatidylcholine (DOPC), (unsaturated lipid) on formation of IF liposomes. Figure 11 A shows the internal volume of IF liposomes containing varying amounts of DOPC by ¹⁴C sucrose encapsulation and TEMPONE EPR methods (open squares and closed squares, respectively). Figure 11 B shows the lipid recovery following the formation of IF liposomes containing varying amounts of DOPC.

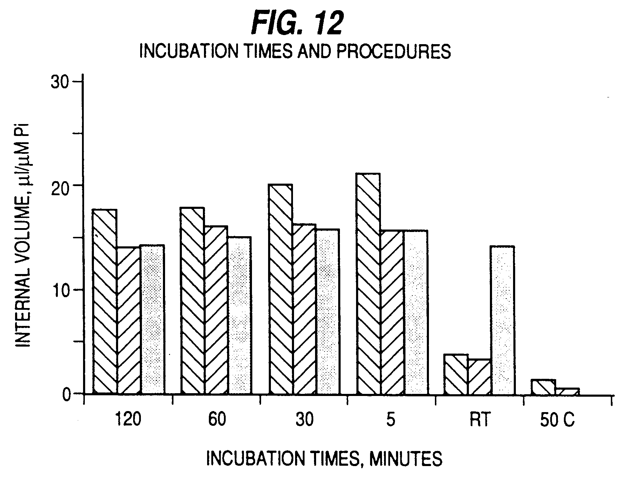

Figure 12 is a histogram demonstrating the effect of lipid incubation time above and below the DPPC Tm (5 minutes, 30 minutes, 60 minutes and 120 minutes), and incubation procedure (room temperature "RT", or 50°C), on the resulting internal volume of the IF liposomes. Throughout this application, the term "PM temp" means 25°C, unless otherwise specified.

### Detailed Description of the Invention

For purposes of clarity, throughout the discussion of the present invention, the following definitions will be used:
"Interdigitation" and "interdigitated" are used throughout the specification to describe a lipid bilayer in which the acyl chain region of one lipid in a bilayer interpenetrates into the other layer of the lipid bilayer. The term interdigitation shall include full interdigitation, mixed interdigitation and partial interdigitation. Full interdigitated liposomes include interdigitated liposomes in which the acyl chains of the lipid interpenetrate fully or partially across the width of the lipid bilayer as in Figure 1A(D). Mixed interdigitated liposomes include interdigitated liposomes in which certain acyl chains of unsymmetrical phospholipids, generally the longer acyl chains, extend completely across the bilayer span, whereas the shorter chains meet end to end in the bilayer midplane as in Figure 1A(C). Another example of mixed interdigitation liposomes includes liposomes in which regions of the liposome are either fully or partially interdigitated and may co-exist with regions that are not interdigitated. Partially interdigitated liposomes include interdigitated liposomes in which the acyl chains of unsymmetrical phospholipids pair such that the longer acyl chain of one bilayer pairs with the shorter acyl chain of the other bilayer as in Figure 1A(B).
"Inducer" is used throughout the specification to describe molecules, including amphipathic molecules of limited size which localize at the lipid bilayer aqueous phase interface region of a liposome and produce an interdigitation-fusion gel which may also be a liquid, of the present invention. The term also contemplates lipids and/or solutes such as bioactive agents that may act as "self-inducers". Hydrostatic pressure may also be an inducer.
"Interdigitation-fusion gel" (IF gel) is used throughout the specification to describe the product that results when an inducer is combined in sufficient quantity to fuse sized liposomes. The resulting sheets of lipid are fused gels for purposes of the present invention and may include products of varying viscosity including liquids, gels and in certain cases, even very viscous products approaching the solid state,
"Interdigitation-fusion liposome" (IF liposome) is used throughout the specification to describe the liposome that results from IF gels which are generally but are not necessarily raised above the lipid transition temperature ("Tm"), but in any case are incubated at a temperature to produce IF liposomes. The inducer may additionally but not necessarily be removed from the interdigitation-fusion gel. In certain embodiments in which the liposome contains self-inducing lipid or the solute is an inducer, the inducer is not removed. The IF liposomes may contain large concentrations of solute such as bioactive agents in bioactive agent:lipid ratios of 1:10 to 15:1.
"Solute" is used throughout the specification to describe any chemical, including buffers and solvents that may be entrapped by the IF gels and liposomes of the present invention. Solutes may include buffers, salts, toxins, microbes and bacteria, pesticides, insecticides, herbicides, fungicides, emulsifying agents, cosmetics, unicellular organisms and a large number of chemical agents, especially including bioactive agents.
"Bioactive agent" is used throughout the specification to describe any agent such as chemical agents which exhibits biological activity when administered to living organisms, including plants, animals such as mammals, and especially including humans. Bioactive agents include drugs and nutrients, as described hereinabove and following.
"Saturated lipid" is used throughout the specification to describe a lipid that may be used to produce the interdigitation-fusion gels and liposomes of the present invention. The term saturated lipid includes, lipids having symmetrical and/or asymmetrical acyl side chains which are saturated, i.e., contain no double bonds, lipids having unsaturated side chains in which the unsaturated carbon-carbon double bonds are oriented in the trans configuration and certain lipids having unsaturated side chains in which the unsaturated carbon-carbon double bonds are oriented in the cis configuration, or mixed fatty acid lipids such as for example SOPC and POPC.
"Interdigitation-fused lipid containing composition" is used to describe the IF gels and liposomes of the present invention.

The present invention relates to lipid containing compositions comprising a sized liposome, 0.4 µm or less, preferably 0.05 µm or less and more preferably 0.025 µm in diameter or less, and an amount of an inducer effective to fuse the liposomes in combination with a solute. The initial liposomes may alternatively be FAT MLVs. In certain embodiment& the solute is a bioactive agent. The compositions of the present invention may advantageously include bioactive agent at high concentrations, for example at bioactive agent:lipid ratios of 1:10 to 15:1.

In the present invention, the sized liposomes which give rise to IF gels and liposomes of the present invention are preferably formed from zwitterionic, cationic, and anionic lipids and phospholipids comprising fatty acyl chains, having 12 to 35 carbon atoms, also including therein saturated (disaturated and partially saturated) and unsaturated and polar or apolar lipids and phospholipids.

For example, the saturated lipids of the invention include but are not limited to for example, dimyristoylphosphatidylcholine, distearoylphosphatidylcholine, dipalmitoylphosphatidylcholine, dimyristoylphosphatidylserine, dipalmitoylphosphatidylserine, distearoylphosphatidylserine, dimyristoylphosphatidylethanolamione, dipalmitoylphosphatidylethanolamine, distearoylphosphatidyethanolamine, dimyristoylphosphatidic acid, distearoylphosphatidic acid, dipalmitoylphosphatidic acid, dimyristoylphosphatidylinositol, distearoylphosphatidylinositol, dipalmitoylphosphatidylinositol, hydrogenated soy phosphatidylcholine, hydrogenated soy lecithin, dipalmitoylphosphatidylglycerol, di-0-hexadecylphosphatidylcholine, dlpalmitoylphosphatidylglycerol, distearoylphosphatidylglycerol, dimyristoylphosphatidylglycerol.

Other saturated lipids include but are not limited to the saturated lipids having symmetrical and/or asymmetrical acyl side chains which are saturated, i.e., contain no double bonds, lipids having unsaturated side chains in which the unsaturated carbon-carbon double bonds are oriented in the trans configuration and certain lipids having unsaturated side chains in which the unsaturated carbon-carbon double bonds are oriented in the cis configuration, or mixed fatty acid lipids such as, for example, SOPC and POPC.

Other lipids for inclusion with the saturated symmetrical lipid are other liposome forming lipids including, for example synthetic or natural phospholipids including mixed chain compositions, for example, phosphatidylcholines (PC), phosphatidylethanolamines (PE), phosphatidylserines (PS), phosphatidylglycerols (PG), phosphatidic acids (PA), phosphatidylinositols (PI), sphingomyelins (SPM) and cardiolipins, among others, either alone or in combination.

In addition to the above lipids, additional lipids including various lysolipids, for example, n-octadecyl-2-methylphosphatidylcholine, n-octadecylphosphatidylcholine, 1-laurylpropanediol- 3-phosphocholine, erythro-N-lignoceroylsphingophosphatidylcholine, cholesterol, water soluble derivatives thereof such as for example, cholesterol hemisuccinate, alpha tocopherols, water soluble derivatives thereof such as tocopherol hemisuccinate, and gangliosides, glycolipids, and glycosphingolipids which may also be included in compositions of the present invention. One of ordinary skill in the art will recognize that the amount and type of lipid which may be included in compositions of the present invention may be varied within the teachings of the present application to produce the compositions according to the present invention.

In the compositions of the present invention, the sized liposomes containing significant quantities of at least one saturated lipid are interdigitated-fused with addition of an inducer. The sized liposomes of the present invention generally containing a saturated phospholipid will undergo full, partial or mixed interdigitation in combination with an effective amount of the interdigitation inducer. While not being limited by way of theory, it is believed that the inducer may function to displace some of the headgroup-associated water molecules and in general, causes an increase in the headgroup surface area. It is preferred that the lipids chosen should undergo full interdigitation in the presence of the inducer; however, it is to be recognized that lipids which provide less than complete interdigitation, i.e., either mixed or partial interdigitation are also contemplated and are within the scope of the present invention. Exemplary interdigitation inducers for use in the present invention include, for example, short chain alcohols including methanol, ethanol, propanol, isopropanol and n-butanol, polyols such as glycerol and ethylene glycol, anaesthetics such as chlorpromazine, tetracaine, phenylethanol, benzyl alcohol and phenylbutanol, among others, buffers such as Tris and chaotropic salts such as thiocyanate SCN-, as well as others referred to hereinabove.

In certain cases, the saturated lipid used to form the IF gels and liposomes of the present invention are self-inducers, i.e., these lipids will interdigitate and form IF gels and liposomes by mixing the lipid in the presence of solute at varying temperatures without the need to add a chemical inducer (for example, DHPC). In addition, in certain cases extremely high pressure may be used to produce interdigitation without the need to include an inducer.

In general, the compositions of the present invention include an amount of an inducer effective for fusing the sized liposomes. The amount and type of inducer utilized to produce liposome fusion will vary as a function of the type of liposome utilized. In general, however, the amount of inducer used comprises about 1.0% to about 50% of the total weight of solution which includes a combination of the sized liposomes, inducer and solute. One of ordinary skill in the art will recognize to readily vary the concentration of the inducer within the teachings of the art and the present application to produce interdigitation gels and liposomes of the present invention.

While not being limited by way of theory, it in believed that sized liposomes fuse into lipid sheets (gels) at certain concentrations of inducer in order to relieve bilayer strain imposed by a small radius of curvature (See, for example, Figure 3). The resulting interdigitation-fusion gel that is produced may capture a high concentration of solute. This includes encapsulating substances which otherwise cannot be entrapped in high solute lipid ratios in liposomes. According to the method of the present invention, when the IF gels are exposed to temperatures usually but not necessarily above their L beta I-L alpha transition temperature ("Tm"), but in any case at a temperature which changes the material properties of the mixture such that IF liposomes are formed, and the inducer is preferably (but not necessarily) removed, liposomes of high captured volume result. These liposomes may vary in size as a function of the solute, liposome and inducer utilized, but generally will range in size from 100 um and more preferably 20 µm to 0.025 µm.

While not being limited by way of theory, it is believed that interdigitation, which renders the lipid bilayer less susceptible to perturbation during liposome formation, can be utilized to capture substances which normally interact with membranes and are difficult to entrap. For example, interdigitation-fusion liposomes of the present invention have been used to entrap high concentrations of aminoglycosides which are very difficult to entrap in high concentrations because of their tendency to interact with membranes. IF liposomes have been shown to entrap gentamicin at a drug/lipid ratio of 1:2 (w:w) whereas typically modified small plurilamellar liposomes (SPLVs) entrap gentamicin at a drug/lipid ratio of 1:10 (w:w).

The production of interdigitation-fusion gels and liposomes of the present invention involves the initial formation of sized liposomes 0.4 µm in diameter or less, more preferably 0.05 µm or less and most preferably no greater than 0.025 µm. Alternatively, FAT MLVs can be used, and in some cases, larger liposomes can be used. Any of the methods available in the art for producing sized liposomes may be utilized including the methods described in greater detail hereinbelow. Typically, liposomes can initially be prepared by vacuum drying a solution of lipid in organic solvent, for example, chloroform, to a thin film in a round bottom or other suitable flank or vessel, followed by hydration of the lipid film with an aqueous solvent such as for example, aqueous buffer or saline solution. Alternatively, liposomes can be formed from admixture of dry lipid powder and aqueous solvent, preferably for example saline solution or aqueous buffer.

The liposomes are then sized according to any methods known in the art such as sonication, extrusion or homogenization, and further described hereinbelow. After the formation of sized liposomes, the solute, preferably a bioactive agent that is to be encapsulated, is generally mixed in the aqueous solvent. Two approaches are generally used to entrap solute depending upon whether or not the solute interacts with liposomes. In the case where the solute does not interact with the liposomes, the solute may be mixed in with the aqueous or aqueous/buffer solvent after formation of the sized liposomes which are to undergo interdigitation. In the case of solute that interacts with the liposomes, the solute is generally mixed in with the aqueous solvent after the formation of interdigitation-fusion gels.

Of course, one of ordinary skill in the art will recognize that the order in which the individual components of the IF gels and liposomes of the present invention are added may vary and is dependent on the type of solute to be entrapped and the type of saturated lipid utilized.

The final concentration of the lipid used to encapsulate solute of the present invention will vary as a function of the concentration and type of the solute desired as well as the type of lipid used, but in general, the weight ratio of drug to lipid in the aqueous solvent will range from 50:1 to 1:100 with the final concentration of lipid falling within the range of 5 to 100 mM. The final weight ratio of drug to lipid in the interdigitation-fusion gels and liposomes of the present invention ranges from 1:10 to 15:1.

After the sized liposomes are formed, inducer is added to the aqueous solvent. The amount of inducer added generally ranges from 1.0% by weight (of the combined weight of lipid, solute and inducer) up to 50 percent by weight. Where ethanol is used as the inducer, the amount of ethanol included is generally 5% by weight (1.0 M) to 20% by weight (4.0 M) and in the case of glycerol the amount of inducer utilized may be as such as 90-100% by weight. The amount of other inducers to be included will vary. In the case of ethanol the final ethanol concentration falls within the range of 0.50 to 10.0 Molar and preferably is within the range of 1.75 to 4.0 Molar.

The presence of inducer in an effective amount will cause the sized liposomes to fuse, resulting in fused sheets of lipid. The IF gel produced by this method may be used topically or for oral administration, for example, as formulations encapsulated in soft gelatin or other oral dosage forms. Alternatively, the gel may be further modified to produce the IF liposomes of the present invention.

To produce IF liposomes of the present invention, the mixture is incubated at a temperature for a period of time sufficient to form a gel. Typically thin period ranges from 1 minute to 1 hour. Thereafter, the temperature is generally but not necessarily raised above the Tm of the lipid for a period of about 1 minute to 1.0 hour. The incubation temperature required may be the Tm of the mixture but is that temperature for any given mixture of lipid, solute or inducer which produces a change in the material properties of the mixture, thereby producing the IF liposomes of the invention. While maintaining this incubation temperature, the inducer may be removed by evaporation (especially in the case of alcohol inducers), positive pressure nitrogen (e.g., N₂ sparge consisting generally of bubbling N₂ through the mixture) or by dilution. This produces IF liposomes varying in size generally between 0.025 and 100 um, more preferably .025 to 20 µm. Unencapsulated drug may be removed from the solvent, if desired. The IF liposomes produced by the above method may be further size reduced to produce liposomes varying or homogeneous in size.

In addition to extrusion, initial liposomes (prior to addition of inducer) for the IF gel or liposome method, and resulting IF liposomes may be size reduced by sonication or homogenization. Sonication employs sonic energy to disrupt or shear the larger liposomes which will spontaneously reform into small liposomes. See, for example, Chapman, et al., BBA, 163, 255 (1968). Sonication is achieved by immersing a glass tube containing the liposome suspension into the sonic epicenter produced in a bathtype sonicator. Alternatively, a probe type sonicator may be used in which the sonic energy is generated by vibration of a titanium probe which is in direct contact with the liposome suspension.

With homogenization the shear forces which break down larger liposomes into smaller ones are generated by, for example, rotorstator type devices such as the Polytron (Brinkman Instruments Co., Westbury, New York, USA), a stator-stator type device such as the Microfluidizer (Microfluidics Corp., Newton, MA, USA), or any number of other such devices which are commonly used to disrupt cells. Due to the fact that all of the above methods involve disruption of the IF liposomes, entrapped solute will be lost when IF liposomes are subjected to any of these procedures. The loss may be minimized however, if the unentrapped solute is not removed from the liposome suspension before size reduction of the liposomes.

A number of other techniques may be used for producing sized liposomes which are to undergo interdigitation-fusion, and for producing sized IF liposomes after the process is complete. These methods include reverse-phase evaporation, infusion procedures, homogenization, sonication, microfluidization and detergent dilution or a combination of these methods. A review of certain of these and other methods for producing liposomes can be found in the text Liposomes, Marc J. Ostro, ed., Marcel Dekker, Inc., New York, 1983, Chapter 1. Sized liposomes may also be produced by an extrusion process.

In the extrusion process, to produce sized liposomes, the liposomes are passed through filters having pore sizes generally ranging from 30 nm to 1 µm to produce liposomes ranging in size from 30 nm to 1 µm in diameter. Preferably, the pore size of the filters through which the liposomes may be extruded ranges from 100 nm to 1 µm. The filters are generally made of polycarbonate, but the filters may be made of any durable material which does not interact with the liposomes and which is sufficiently strong to allow extrusion under sufficient pressure. Preferred filters include "straight through" filters because they generally can withstand the higher pressure of the preferred extrusion processes of the present invention. "Tortuous path" filters may also be used. In the preferred embodiments of the present invention, pre-IF fusion liposomes are extruded through 50 to 100 nm polycarbonate filters to produce liposomes having a diameter of 50 to 100 nm.

Any extrusion process available in the art may be used to produce sized liposomes which will undergo interdigitation-fusion. The extrusion process may be performed sequentially or once under high pressure. Particularly preferred extrusion processes for use in the present invention include those disclosed in Cullis, et al., PCT Application PCT/US85/01161, Publication Number WO 86/00238 entitled "Extrusion Techniques for Producing Liposomes", published January 16, 1986.

Other methods for sizing the liposomes of the invention either before or after fusion are filtration methods employing asymmetric filters such as for example, Anotec^{R} filters according to commonly-assigned copending U.S. Patent application entitled "Liposome Extrusion Process", U.S. Serial No. 593,200, filed October 5, 1990, and WO92/05772 which involves extruding liposomes through a branched pore type aluminum oxide porous filter.

Alternatively , the liposomes can be sized using a homogenization or milling procedure such as a colloid mill for example the Gifford Wood colloid mill. The liposomes may be passed one or more times through the mill until the appropriate size and homogeneity is achieved, analyzed for size distribution using either the Nicomp Particle sizer or the Malvern Particle sizer. The liposomes, alternatively, may be passed through a Microfluidizer device, discussed hereinabove, which likewise homogenizes the liposomes.

If desired, the resulting liposomes can be separated into populations using any methods known in the art for so separating; such a process is, for example, tangential flow filtration. This process as used for the separation of liposomes according to size is disclosed in commonly assigned and copending U.S. Patent application entitled "Method for Size Separation of Particles", Serial No. 225,327, filed July 28, 1988 and WO89/00846.

In this procedure, a heterogeneously sized population of liposomes is passed through one or more tangential flow filters thereby resulting in a size distribution with in upper and/or lower size limit. For example, when two filters of differing sizes are employed, for example, a first filter of 5 µm pore size, liposomes less than 5.0 µm pass through the filter and into the filtrate, which is then passed through a second filter of smaller pore size, for example, 2.0 µm pore size. In this case, the retentate contains liposomes of a homogeneous size distribution having discrete size limits of 5.0 and 2.0 µm. Filters of alternative pore size may be employed to result in discrete populations having upper and lower size limits.

The liposomes which undergo interdigitition-fusion in the presence of an inducer preferably are 0.4 µm in diameter or less, more preferably 0.05 µm or less, and most preferably are 0.025 µm or less in diameter. Alternatively, the initial sized liposomes of the invention may be FAT MLVs. The IF liposomes which are produced by the general method of the present invention generally range in size from 100 um but more preferably 20 µm to 0.025 µm, and generally in the range of 2 to 20 µm. These resulting IF liposomes may be further size reduced to produce liposomes of varying sizes by sonication, homogenization and extrusion techniques described hereinabove. It should be noted, however, that although these IF liposomes of the present invention may be down sized, the down sizing often results in the loss of bioactive agent from the liposomes. Thus, IF liposomes which undergo further down-sizing, for example, by the previously discussed extrusion process or other processes such as sonication and homogenization to vary the size of the liposomes produced, may encapsulate diminished concentrations of bioactive agents.

Bioactive agents for use in the present invention may include vitamins, hormonal agents, anti-metabolites, anti-microbial agents, antifungal agents, antibiotics, proteins, peptides, ribo and deoxyribonucleic acids, nucleotides, nucleosides, oligonucleotides, antihistaminic agents, neuropharmacologic agents including sedatives and hypnotics, steroidal and nonsteroidal antinflammatory agents, diuretic agents, antihypertensive agents, antiarrhythmic agents, immunogens, immunomodulators, contraceptive agents, radiographic contrast agents, NMR contrast agents, antiviral agents and vascular dilating agents, among others. In certain preferred embodiments of the present invention, radiocontrast agents, NMR contrast agents, peptides and naturally occurring, synthetic and semi-synthetic antimicrobial agents, for example, cephalosporins and aminoglycosides are utilized in the present invention. Exemplary radiocontrast agents for use in the present invention include, for example, iohexol, iopamidol, ioxoglate, iotrolan, ioversol, iothalamate, iodimide, iodipamide, iopromide, iopentol, iodixanol, metrizamide, mixtures thereof and their pharmaceutically acceptable salts. Exemplary aminoglycosides include gentamicin, tobramycin and amikacin.

Suitable biological agents for use in the present invention include any agent which exhibits favorable biological activity when administered topically or systemically and is stable to the compositions of the present invention. Agents which may be topically administered for their affect on the skin include salicylic acid, resorcinol, phenol, retinoic acid, and their equivalents. Other agents for use in the present invention include certain desensitizing agents, for example antigens and vaccines, vitamins, nutrients, such as amino acids, essential fats and minerals, retinoids, anti-neoplastic and anti-tumor agents, including certain alkylating agents.

Additional bioactive agents for use in the present invention include the benzodiazepines, antipyretic agents, antispasmodics, antipruritic agents, sympathomimetics, decongestants, tranquilizers, antispasmodics, cardioactives, other cardiac agents, anti-emetics, sedatives and hypnotics, steroidal agents, progestational agents, local anesthetics and antibiotics. Other antimicrobial agents may also be used in the present invention including antifungal agents.

The above-listed group of bioactive agents, among other agents, including their pharmaceutically acceptable salts, are contemplated for use in the present invention. Determination of compatibilities of the above listed agents with and the amounts to be utilized in compositions of the present invention are within the ordinary skill in the formulation art. The stability and applicability of individual pharmaceutical agents are well within the ordinary skill of practitioners in this art.

It will be appreciated that the actual preferred amounts of bioactive agent utilized in a specific case may vary according to the severity of a pharmacological or disease condition and the expected pharmacokinetics of bioactive agent in the individual patient. Dosages for a given host can be determined using conventional considerations, e.g., by customary comparison of the differential activities of the subject bioactive agent by means of an appropriate, conventional pharmacological protocol.

The IF liposomes and gels of the present invention may be administered to any animal including mammals, such as humans. For administration to humans in the treatment of afflictions, the prescribing physician will ultimately determine the appropriate dose for a given human subject, and this can be expected to vary according to the age, weight, and response of the individual as well as the nature and severity of the patient's symptoms. The present invention provides a readily available method to allow wide variations in liposomal drug concentrations.

The mode of administration of compositions of the present invention may determine the sites in the organism to which the compositions will be delivered. For instance, delivery to a specific site of infection may be most easily accomplished by topical application (if the infection is external, e.g., on areas such as the eyes, skin, in the ears or on afflictions such as wound or burns) or by absorption through epithelial or mucocutaneous linings (e.g., nasal, oral, vaginal, rectal, gastrointestinal, mucosa, etc.). Such topical application may be in the form of creams or ointments. The interdigitation-fusion gels of the present invention are preferably used topically. However, the IF gels of the present invention may be used orally in formulations in which the lipid, upon contacting the fluids of the mouth or gastrointestinal tract forms a liposome in situ.

The IF liposomes containing bioactive agent may be administered alone but will generally be administered in admixture with a pharmaceutical carrier selected with regard to the intended route of administration and standard pharmaceutical practice, thereby forming pharmaceutical compositions. The IF liposomes of the present invention may be injected parenterally, for example, intravenously, intramuscularly, or subcutaneously. For parenteral administration, these liposomes are best used in the form of a sterile aqueous solution which may contain other solutes, for example, sufficient salts, glucose or dextrose to make the solution isotonic.

For the oral mode of administration, the liposomes of the present invention can be used in the form of tablets, capsules, lozenges, troches, powders, syrups, elixirs, aqueous solutions and suspension. In the case of tablets, carriers which can be used include lactose, sodium citrate, and salts of phosphoric acid. Various disintegrants such as starch, and lubricating agents, for example, starch, may be used. For oral administration in capsule form, useful diluents are lactose and high molecular weight polyethylene glycols. When aqueous suspensions are required for oral use, certain sweetening and/or flavoring agents can be added.

Bioactive agents for use in the present invention may include those listed hereinabove, and include their pharmaceutically acceptable salts. Determination of compatibilities of the above listed agents with and the amounts to be utilized in compositions of the present invention are within the ordinary skill in the formulation art. The stability and applicability of individual pharmaceutical agents are well within the ordinary skill of practitioner in this art. It will be appreciated that the actual preferred amounts of bioactive agent utilized in a specific case may vary according to the severity of a pharmacological or disease condition and the expected pharmacokinetics of bioactive agent in the individual patient. Dosages for a given host can be determined using conventional considerations, e.g., by customary comparison of the differential activities of the subject bioactive agent by means of an appropriate, conventional pharmacological protocol.

IF liposomes can be remote loaded, for example, to incorporate bioactive agents. If desired, IF liposomes can be dehydrated using, for example, the procedures of Janoff et al. U.S. Patent No. 4,880,635.

The following examples are provided to illustrate the present invention and should not be construed to limit the scope of the invention of the present application in any way.

### EXAMPLE 1

Liposomes comprising dipalmitoylphosphatidylcholine (DPPC, obtained from Avanti Polar Lipids, Birmingham, Alabama, USA) were formed in 1 ml of an aqueous buffer solution to a concentration of 20mM DPPC and additionally containing 0.04 mM diphenylhexatriene (DPH, purchased from Molecular Probes, Eugene, Oregon, USA). After formation of the liposomes, ethanol was added to a final concentration of 0.3 M to 2.5 M of the aqueous solution.

DPH fluoresces maximally when incorporated into the liposome bilayer. Interdigitation results in the reorientation of DPH from the bilayer membrane with a concomitant decrease of fluorescence. As shown in Figure 2, interdigitation is greater where higher concentrations of ethanol are present, for all liposomes. The effect of interdigitation by the same amount of ethanol is greater in those liposomes having a larger diameter. In Figure 2, Fo = DPH fluorescence in the absence of ethanol; F = DPH fluorescence in the presence of ethanol. Excitation = 351 nm. Emission was detected between 380 and 580 nm and quantitated by weighing.

### EXAMPLE 2

### Lipid Mixing of Liposomes

Lipid mixing of sized DPPC liposomes was determined as a function of the size of the liposomes and concentration of inducer. Liposomes comprising DPPC were formed in an aqueous buffer solution containing 20 mM DPPC. A marker population of liposomes containing 99% by weight DPPC, 0.35% by weight N-benzyldiphosphatidylethanolamine (NBD-PE) and 0.65% by weight rhodamine-phosphatidylethanolamine were formed in 1 ml. of an aqueous buffer solution. These probes form a donor-acceptor pair. The NBD moiety is excited at 465 nm and via resonance energy transfer (RET) becomes quenched by the rhodamine acceptor which itself becomes excited in a distance dependent phenomenon. These liposomes were mixed with blank liposomes at a 1:10 ratio. Emission spectra were recorded between 480 and 680 nm. Lipid mixing in DPPC liposomes of varying size as a function of ethanol concentration can be determined by the loss of RET from the NBD moiety to the rhodamine moiety. A standard curve was generated by preparing liposomes of 0.35 mole percent NBD-PE and 0.65 mole percent rhodamine-PE with sequentially decreasing these mole percents to 0.035 and 0.065 respectively. A direct comparison from the 1/10 mixing experiments with this standard curve indicates the degree of lipid mixing, an indication of membrane fusion.

### EXAMPLE 3

### Comparison of Trapped Solute in Various Vesicle Types

A number of liposomal formulations were prepared. The amount of trapped aqueous phase was determined and compared for each "type" of liposome prepared. The results appear in table 1, below.

For the preparation of IF liposomes, MLVs were prepared as described below to a final concentration of 20 umoles DPPC per ml of aqueous buffer. The MLVs were then sonicated in a bath type sonicator at 50°C until translucent (SUVs). After the SUVs cooled to room temperature, ethanol was added to a final concentration of 2.0 M in the final aqueous suspension. For examples A1 and A2 (see table 1, below), ethanol was removed by dilution followed by washing. For B1 and B2, ethanol was removed via positive pressure displacement using N2, after which the samples were diluted and washed. Sample C had one half the initial lipid concentration of A and B and ethanol removal was achieved as for sample A.

For the preparation of MLVs, 100 mg DPPC in 5 ml chloroform was rotary evaporated to a thin dry film in a round bottom flask to which a 1 ml aqueous buffer solution containing 0.04 mM diphenylhexatriene was added. Thereafter, the lipid mixture was vigorously vortexed until all lipid was removed from the wall.

FATMLVs were formed by subjecting unwashed MLVs as described above to 5 freeze and thaw cycles as described by Bally et al., U.S. Patent No. 4,975,282, issued December 4, 1990.

SPLVs were prepared by forming the thin dry film of DPPC as described above for MLVs and then dissolving the lipid film in 5 ml ethyl ether to which 0.5 ml of aqueous buffer was also added. This mixture was then emulsified in a bath type sonicator, a stream of N2 was used to stir the emulsion while removing the ether as described by Lenk, et al., U.S. Patent No. 4,522,803. Ether removal was continued until no residual odor was detected (approximately five minutes). The resulting lipid mixture was resuspended in 1 ml aqueous buffer.

MPVs were formed as described in U.S. Patent No. 4,588,578, by preparing a monophase of 100 mg DPPC and 5 ml chloroform, 5 ml ethanol and 0.5 ml aqueous buffer, rotary evaporating to dryness, and resuspending the suspended film in 1 ml aqueous buffer by vigorous vortexing.

To determine the captured aqueous volume, 20 µl of a 10 mM 4-trimethylammonium TEMPO (4-TMAT) solution was added to 0.98 ml of the liposomal suspensions. The samples were then vortexed and the outer aqueous phase was separated from the liposomes by centrifugation. Because 4-TMAT neither binds to nor permeates the liposomes used in this study, it is concentrated in the outer aqueous phase. Measurement of 4-TMAT's concentration allows for calculation of the internal aqueous phase or captured volume as detailed in Perkins, et al., BBA, 943, 103 (1988). The results of this analysis appear in Table 1, below. As seen, the IF liposomes sequester significantly greater volumes, in some cases as much as 10 times that attained with the other liposome types.

**Table 1**

| Comparison of Trapped Solute | | |
|---|---|---|
| Liposome Type | | *Captured Volume (µl/µmole) |
| IF | A1 | 6.7 |
| | A2 | 7.8 |
| | B1 | 8.3 |
| | B2 | 7.2 |
| | C | 8.9 |
| MLV | 1 | 0.56 |
| | 2 | 0.78 |
| MPV | 1 | 0.71 |
| | 2 | 1.9 |
| SPLV | 1 | 2.0 |
| | 2 | 2.8 |
| FATMLV | 1 | 2.7 |
| | 2 | 2.6 |

| | | |
|---|---|---|
| *Captured volumes were measured using the EPR technique (Perkins, et al. (1988) Biochim. Biophys. Acta 943, 103) where the samples are examined after formation. | | |

### EXAMPLE 4

### Procedure for Formation of IF Liposomes

Liposomes (LUVs, MLVs, SPLVs, etc. as prepared above) comprising a saturated lipid such as dimyristoylphosphatidylcholine, dipalmitoylphosphatidylcholine or distearoylphosphatidylchoine are prepared and sized to 0.4 microns or less (preferably, 0.025 microns) by extrusion, sonication or homogenization. A bioactive agent which does not interact with the lipid is then mixed in with the aqueous solvent used to form the liposomes (final lipid concentration should be about 5 to 100 mM, preferably about 10 to 35 mM). The temperature of the lipsomes are below the main phase transition temperature of the lipid. Thereafter, ethanol is added to a final concentration of about 1.75 to about 2.5 M in the aqueous solvent. In the case of bioactive agents which interact with the lipid, the agents are generally added to the solvent after the addition of ethanol and formation of the IF gel. At this stage, the procedure can be stopped and the resulting gel used in topical and oral formulations. Alternatively, the gel may be used to form IF liposomes of the present invention.

To form IF liposomes, the gel is incubated at a temperature below the Tm of the lipid for a period ranging from about 1 minute to about 1 hour followed by an incubation period of about 1 minute to about 1.0 hour at a temperature above the Tm of the lipid. The inducer is then removed by evaporation, positive nitrogen pressure or dilution. In the case of ethanol, the ethanol may be diluted to a concentration below about 0.2 M. As the inducer is removed, liposomes form, generally varying in size from about 0.25 microns to about 20 microns.

### EXAMPLE 5

### Scale-up of Diatrizoate-DPPC IF Liposomes

600 mg of DPPC were dried to a film from chloroform by rotary evaporation, then dried further under vacuum for 16 hours. The lipid was resuspended in 6 ml of 0.9% saline by incubation in a 51°C bath for approximately 15 minutes. The resulting multilamellar liposomes (MLVs) were transferred to a 30 ml Corex tube and bath sonicated for two hours at 51°C until the solution was translucent. At this point only 4.1 ml of liposome suspension could be retrieved. 12 ml of diatrizoate (Renografin-76^{tm}, available from Bristol-Myers Squibb), 12 ml of deuterated water ("dH₂O"), and 2 ml of ethanol were mixed in a 50 ml of centrifuge bottle to which the 4.1 ml lipid (still at 51°C) was added and then briefly vortexed. Within 10 minutes the mixture became a loose, pourable gel which was allowed to set at room temperature for 2 hours. Next, the suspension was incubated in a 51°C bath for 1 hour. At this point the sample was split in half to facilitate ethanol evaporation. While still immersed in the bath, N₂ was bubbled through each aliquot for a period 12 minutes. The samples were allowed to cool to room temperature followed by dilution with 20 ml of 0.9% saline per aliquot. The preparation was washed by repetitive centrifugation at 10,000 x g at 20 C for 10 minutes for 3 cycles. The sample was assayed as described for Example 6 above. The results appear in Table 2, below.

**Table 2**

| Aliquot | I:L | % Entrapped | mg/ml Iodine | Pellet Weight (mg) |
|---|---|---|---|---|
| A | 2.4 | 16%¹ | 48.8 | 7.42 |
| B | 3.8 | 25% | 67.6 | 8.07 |

| | | | | |
|---|---|---|---|---|
| 1- Based on 12 ml. of diatrizoate initially added. | | | | |

### EXAMPLE 6

### Diatrizoate-HSPC IF Liposomes

400 mg HSPC (Natterman Phospholipids) was hydrated at 70 C in 10 ml dH₂O for about 1 hour and probe sonicated until translucent (30 minutes). 14 ml diatrizoate (Squibb) containing approximately 1 µCi/ml 125I-diatrizoate was mixed with 2.1 ml dH₂O and 4.9 ml ethanol in a 50 ml screw-top Corex^{Tm} tube. 7 ml of the HSPC small unilamellar liposomes (SUVs) were added while mixing; this was done while the SUVs were still above their Tm. A solid gel formed immediately and was allowed to set at room temperature, covered, for approximately 1 hour. The preparation was then incubated for 2 hours in a 70°C water bath and uncovered after which N₂ was bubbled through the now liquid prep for 23 minutes. The N₂ flow rate was at 10 on the Manostat^{Tm} flowmeter. A 4 ml aliquot was dispensed into a 30 ml Corex^{Tm} tube and allowed to cool to room temperature . 10 ml of 900 mOsm meglumine buffer (prepared from meglumine, NaCl, citrate, EDTA) was added and the preperation was vortexed briefly. The unentrapped diatrizoate was removed by repetitive centrifugation at 10000 X g at 18°C for 15 minutes for 3 cycles. The final iodine concentration was determined by extrapolation from a UV spectrophotometric assay of diatrizoate (A256) to be 106.3 mg/ml. The final lipid concentration was 12.1 mg/ml as determined by the standard methodologies described by Chen, et al., Analytical Chem., 28, 11, 1756 (1956). The final I:L ratio was 8.8 (w/w). The resulting liposomal suspension was stored under ambient conditions.

### EXAMPLE 7

### Iotrolan-HSPC IF Liposomes

1 g HSPC (Natterman Phospholipids) was hydrated at 70°C in 25 mls dH2O for about 1 hour and probe sonicated until translucent (about 30 minutes). The SUVs produced were transferred to a 50 ml screw-top Corex^{TM} tube and spun at 5000 X g for about 5 minutes in order to pellet any titanium present. The SUVs were decanted from the titanium pellet and incubated in a 70°C water bath for about 5 minutes before being added to the following solution: 44 ml ¹²⁵I-labelled Iotrolan was mixed with 15.6 ml ethanol and 6.4 ml dH2O. This solution was then divided into 4 X 16.5 ml aliquots in 50 ml screw-top Corex^{TM} tubes. 5.5 ml of SUVs were added to each tube while mixing resulting in the formation of loose gels. The gels sat covered for 1 hour at room temperature, then each was transferred to a 70°C water bath for 1 hour, uncovered, after which N₂ was bubbled through the liquid in each tube for 13 minutes at a flow rate of 40 on the gas flowmeter. Each tube was emptied into a 250 ml Erlenmeyer flask where it cooled to room temperature. About 150 ml sterile PBS (phosphate buffered saline without Ca and Mg, pH 7) was added while swirling the flask. The unentrapped iotrolan was removed by repetitive centrifugation at 10000 X g for 10 minutes at 18°C for 3 cycles. The final iodine concentration was based on the initial specific activity of the iotrolan solution at 300 mg/ml iodine and was found to be 152.7 mg/ml. The final phospholipid concentration was determined to be 32.7 mg/ml by the method of Chen, et al., Analytical Chem., 28, 11, 1756 (1956) and was corrected for the presence of phosphate in the buffer. These values resulted in a final I:L ratio of 4.7 (w/w). The resulting liposome suspension was stored under ambient conditions.

### Example 8

### Shelf Stability of Radiocontrast Agent IF Liposomes Stored Under Ambient Conditions

50 ul of either diatrizoate or iotrolan HSPC IF liposome preparations (radiolabelled) were diluted with 1 ml of their original suspension buffer and centrifuged at 16,000 X g in a microfuge at room temperature for 10 minutes. The supernatants were removed and both the pellets and the supernatants counted for 125I. After 62 days at 25°C, there was 4% of the radiolabel in the supernatant of the diatrizoate liposomes and after 54 days, 3% in the supernatant of the iotrolan liposomes. Based upon these results, the preparations exhibit a shelf life of at least about 1 year when stored under ambient conditions.

### EXAMPLE 9

### Effect of Initial Lipid Concentration on Entrapment of Diatrizoate in DPPC IF Liposomes

The results which appear in Table 3, below represent IF liposome preparations made as previously described In Example 5 with variation in the initial lipid concentration. Briefly, in 15 ml. Corex tubes, 1 ml diatrizoate was mixed with dH2O and DPPC sized unilamellar Liposomes (SUVs) at 50 mg/ml to achieve the final volumes shown below. Before lipid addition, 87.5 ul ethanol per ml. (final volume 2M) was added to the diatrizoate solution. Preparations sat, capped, for 1 hour at room temperature followed by incubation in a 51°C bath, uncapped. After 1 hour N2 was bubbled through each sample for 2 minutes. After cooling to room temperature, each sample was diluted with 10 ml of 0.9% saline solution and washed by repetitive centrifugation at 10,000g for 15 minutes at 20°C for 3 cycles. The samples were assayed as previously described (Example 3). The results appear in Table 3, below.

**Table 3**

| mg. DPPC/Total ml | I:L | % Entrapped¹ | mg/ml Iodine | pellet Weight |
|---|---|---|---|---|
| 50 mg/2 ml. | 1.7 | 38 | 57.6 | 2.47 |
| | 1.8 | 35 | 58.9 | 2.18 |
| 25 mg/2 ml. | 3.5 | 37 | 68.7 | 2.01 |
| | 3.1 | 28 | 62.1 | 1.66 |
| 25 mg/3 ml. | 5.0 | 49 | 73.5 | 2.47 |
| | 5.6 | 56% | 80.3 | 2.56 |

| | | | | |
|---|---|---|---|---|
| 1- Based on 1 ml diatrizoate initially added. | | | | |

### Example 10

### Entrapment of Gentamicin Via IF Liposomes

A solution of gentamicin sulfate (Sigma Chemicals, St. Louis, Missouri, USA) was prepared in 0.9% saline solution to a final concentration of 500 mg/ml. Separately, 100 mg of DPPC (Avanti Polar Lipids) was evaporated to dryness and hydrated in 2.5 ml saline to a final concentration of 40 mg/ml. The DPPC mixture was sonicated on a bath sonicator to clarity.

The following mixtures were then made. Note that the ethanolic inducer was added before the solutions containing gentamicin sulfate.
A - 0.5 ml. DPPC solution, plus ethanol ("EtOH") (to a final concentration of 2M) and 0.5 ml. Gentamicin solution;
B - 0.25 ml. DPPC solution, plus 0.25 ml 0.9% NaCl saline solution, EtOH (to a final concentration of 2M) and 0.5 ml. of Gentamicin solution;
C - 0.25 ml. DPPC solution, plus 0.75 ml saline, EtOH (to a final concentration of 2M) and 1.0 ml. of Gentamicin solution;

Each of the above preparations were then assayed for gentamicin activity by the agar well diffusion bioassay to determine lipid:gentamicin concentration. In brief, the liposomes in each of the above preparations were then disrupted with 0.2% Triton-X 100 (Biorad Laboratories, Richmond CA) and assayed for gentamicin activity using an agar well diffusion bioassay with Bacillus subtilis (ATCC #6633) as the indicator organism. Lipid concentration was determined by standard methods described by Ames, et al., Journal of Biological Chem. 235, 236, 769 (1960).

The results of the bioassay determinations appear in Table 4, below. The results indicate that very low lipid/gentamicin weight ratios may be obtained.

**Table 4**

| Entrapment of Gentamicin in IF Liposomes | | | |
|---|---|---|---|
| Sample | Gentamicin¹ mg/ml | Lipid mg/ml | Lipid/Gentamicin Weight Ratio |
| A1 | 6.18 | 19 | 3.07 |
| A2 | 3.13 | 12.5 | 4.0 |
| B1 | 2.16 | 13.2 | 6.11 |
| B2 | 3.15 | 11.0 | 3.49 |
| C1 | 4.25 | 8.6 | 2.02 |
| C2 | 2.46 | 6.05 | 2.46 |

| | | | |
|---|---|---|---|
| 1- Corrected for Potency (616 ug/mg). | | | |

### Example 11

### Scale up of DSPC - Iotrolan IF Liposomes

Eight (8) grams of DSPC were mixed in 200 ml of Water for Injection ("WFI") at 70°C for 30 minutes. The resulting suspension was passed through a Microfluidizer homogenizer 25 times at a pressure of 11,000 psi thereby forming SUVs. The resulting SUVs were filtered through a 0.22 µm pore size Millipore tortuous path polymeric filter.

Iotrolan (92 ml, at 300 mg/ml), 13.4 ml WFI, and 32,6 ml ethanol were admixed in a 2,000 ml capacity round bottom flask. The SUVs (44 ml) were admixed in the flask at room temperature (about 25°C) and mixed using a banana paddle mixer for 10 seconds. The gel which formed thereafter mixing was allowed to sit undisturbed for 1.25 hours.

The round bottom flask was placed in a 70°C water bath and mixed using the banana paddle at 66 rpm for one (1) hour. Ethanol was then removed by nitrogen sparge over the aqueous surface at a rate of 4.7 liters N₂/minute for one (1) hour, collecting the ethanol in a trap, with the mixing increased to about 135 rpm. The final volume was adjusted to about 400 ml with carbonate buffer (0.4 mg/ml NaHCO₃, 0.1 mg/ml disodium EDTA, in 0.9% saline). The resulting IF liposomes were washed, thereby separating liposomes from unentrapped iotrolan by diafiltration through a 0.2 um Microgon filtration device. Seven washes of 100 ml were employed with removal of 300 ml last, as a concentrating step. This washing step proceeded for 25 minutes.

Analysis of the resulting iotrolan/DSPC liposomes yielded the following results shown in Table 5, below:

**Table 5**

| | |
|---|---|
| Lipid concentration | 23.0 mM, 18.2 mg/ml |
| Lyso PC content | 0.9% |
| Iotrolan entrapped | 264.8 mg/ml |
| Free Iotrolan | 1.4% |
| Iotrolan/DSPC | 14.5 |
| Captured Volume | 13.7 µl/µmole |
| Size distribution | 90% less than 3.6 µm |
| | 50% less than 2.8 µm |
| | 10% less than 1.2 µm |

### EXAMPLE 12

### Encapsulation Efficiency of DPPC - IF Vesicles as a Function of Ethanol Concentration

DPPC (powder) was mixed with 10 mM Tris HCl, 150 mM NaCl also containing a trace amount of ¹⁴C sucrose, at pH 7.4 to a concentration of 20 mg/ml DPPC, for a total of 2.0 ml. The mixing was done at a temperature above the phase transition temperature of DPPC, at 50°-53°C, and resulted in MLVs. The MLVs were sonicated for one hour at 50-53°C and cooled to room temperature, resulting in SUVs of about 30-50 nm in diameter. To the 2.0 ml of SUVs was added enough ethanol (100%) to result in a 3.0 M ethanol concentration (0.43 ml ethanol; 20% ethanol by weight total); the mixture was vortexed to homogeneity. The suspension was allowed to sit capped and undisturbed for one hour at room temperature, then was incubated one hour at a temperature above the transition temperature of DPPC i.e., at 50°-55°C, with the cap loosened. While still incubating above the lipid Tₘ, a gentle stream of N₂ was bubbled through the mixture for 3 minutes. A sample (100uL) was removed for a ¹⁴C sucrose encapsulation study, and a 4 uL and 8 ul aliquot also removed for determination of Pi according to the Bartlett phosphorous assay of Chen et al. Ten ml of Tris/NaCl buffer was added to the resulting IF liposomes, and the mixture was centrifuged at 9,000 x g for 15 minutes, the supernatant decanted and the pellet resuspended in Tris/NaCl buffer and centrifuged two additional times for a total of 3 washes. The pellet was finally resuspended in 2.0 ml buffer. Another 200 µL sample was removed for the encapsulation study, as well as 4.0 µL and 8.0 µL aliquots for the Pi assay.

The above method was repeated using a total ethanol concentration of 1.0, 2.0, 2.5, 3.5, and 4.0 M in solution.

The internal volume of the IF liposomes is expressed as µL per µmole of phosphorous Pi, and was measured by ¹⁴C sucrose encapsulation and CAT 1 EPR methods.

The ¹⁴C sucrose encapsulation was performed as follows: Following the N₂ bubbling step, the 100µl sample removed for the ¹⁴C sucrose encapsulation study was counted in a Beckman model Ls 6800 scintillation counter. Similarly, following the centrifugation step, the 200 µl sample removed was centrifuged at 3,000 x g using a table top centrifuge, and both the pellet and the supernatant was counted in the scintillation counter. In addition, the Pi was determined as before. The ¹⁴C sucrose encapsulation was thereby determined and the results are represented graphically in Figure 4A.

The CAT 1 EPR study was performed as in Example 13 hereinbelow.

As shown in Figure 4B, the internal volume of the DPPC IF liposomes increased as a function of increased ethanol concentration, consistent with higher encapsulation efficiency of liposomes at higher ethanol concentrations. The percentage of DPPC recovered following the three centrifugation washes is shown in Figure 4C.

### EXAMPLE 13

### Encapsulation Efficiency of DSPC, DHPC, DOPC and EPC IF Vesicles as measured by sucrose and EPR Methods

The methods of Example 12 were repeated using the lipids DSPC, DHPC, DOPC and EPC, using a final concentration of 3.0 M ethanol. The low temperature incubations following ethanol addition were done at 50°C for DOPC and EPC. The high temperature incubation was done at 70°C for all samples.

Incubation of DSPC took place at 70°C, DHPC at 50°C, and DOPC and EPC at 5°C.

The DOPC and EPC samples were not washed by centrifugation but filtered through Amicon 30K microconcentration device (Grace Co.) filters. 100 µl aliquots were removed from the filtrate and from the sample before and after the filtration for the entrapment efficiency analysis.

Entrapment efficiency was calculated by the sucrose encapsulation, CAT 1 EPR and TEMPONE EPR methods, methods for performing all of which are recited hereinbelow.

As shown in Figure 5, the three phosphatidylcholines which are known to interdigitate (DPPC, DSPC and DHPC) all produced IF liposomes with high internal volumes. EPC and DOPC had relatively low internal volumes, were relatively small and could not be pelleted using a table top centrifuge (9,000 x g). Therefore it was not possible to measure internal volumes of these liposomes using the CAT 1 EPR method.

### CAT 1 EPR METHOD FOR DETERMINING ENTRAPMENT

This method is alternatively known as the external solvent volume method, described in Perkins et al., 1988, Biochim. Biophys. Acta, 943:103-107. The internal volume of liposomes was determined by subtracting the external solvent volume, calculated by measuring the concentration of a membrane impermeant spin probe from the total volume of the liposome suspension. The external solvent volume was calculated by adding a known amount of a spin probe 4-trimethylammonium-2,2,6,6-tetramethylpiperidine-1-oxyl iodide ("CAT 1") to a liposome suspension. The liposome suspension was centrifuged to pellet the liposomes. The concentration of CAT 1 in the solvent was determined by comparison of the magnitude of the CAT 1 EPR signal from the supernatant to an EPR signal versus CAT 1 concentration calibration curve. The CAT 1 concentration in the supernatant was higher than what would be expected based on the amount of CAT 1 added because the volume available to the spin probe was reduced as the probe was excluded from the inside volume of the liposomes. Correction was also made for the sample volume due to the lipids themselves.
Procedure: A stock solution containing 10 mM CAT 1, 10 mM Tris HCl (pH 7.4), 150 mM NaCl was used for the calibration buffer solutions containing 100, 200, 300 and 400 µM CAT 1. The EPR spectrum of each stock solution was recorded with a Brucker ER 100D spectrometer. The peak to peak height of the M_{I}=+1 resonance line of each spectrum measured for each concentration and a peak height versus CAT 1 concentration curve was constructed.
CAT 1 stock (0.20 µM) was added to 1.00 ml of the liposome suspension (Vₜ) and vortically mixed. The liposomes were pelleted by centrifugation on a table top centrifuge at 9,000 x g and a small portion of the supernatant was drawn into an EPR capillary tube and sealed. The peak to peak height of the M_{I}=1 resonance line was measured. The solvent concentration of CAT 1 was determined from the magnitude of the sample EPR signal and the calibration curve.
The external solvent volume Vo was obtained by the calibration curve. The external solvent volume Vo is equal to M/C where M is the moles of CAT 1 added and C is the CAT 1 concentration in the supernatant. V1 is the volume occupied by the lipid, is expressed as Vi=1.00-Vt-V1, where V1 is the volume of the lipid. The internal volume, Vi, divided by the phosphate content of the sample gives the internal volume per uM Pi which is the standard way of expressing the internal volume of liposomes.

### TEMPONE EPR METHOD FOR DETERMINING ENTRAPMENT

This method is alternatively known as the broadening agent method, described in Anzai et al., Biochim. Biophys. Acta 1988, 937:73-80. The internal volume of liposomes is determined by measuring the amount of membrane permeant EPR spin probe broadening agent. Normally rapidly tumbling aqueous EPR spin probes have relatively narrow spectral line shapes, however, addition of paramagnetic ions decreases the spin-spin relaxation time (T₂). If the broadening agent is at high enough concentration it can drastically broaden the spectral line shape and dramatically decrease the peak to peak height of EPR signals. In effect, if the EPR broadening agent has access to the spin probe, the probe signal is eliminated.

The measurements are done by adding the membrane permeant EPR spin probe 4-oxo-2,2,6,6-tetramethylpiperidine-1-oxyl ("TEMPONE") to liposomes suspensions. The two 200 µl aliquots were removed from each liposome suspension. One of the aliquots was diluted with 200 µl buffer while the other was diluted with 200 µl of buffer plus the membrane impermeant broadening agent potassium tris(oxalato)chromate (III). The EPR signal from the aliquot diluted with buffer is proportional to the total sample volume, while the EPR signal from the aliquot diluted with the potassium tris(oxalato)chromate (III) is proportional to the sample volume inside the liposomes.
Procedure: A stock solution of 50 mM TEMPONE, 10 mM Tris-HCl (pH 7.4), 150 mM NaCl stock solution was prepared. TEMPONE stock (10 µl) was added to 0.5 ml suspension of liposomes and vortically mixed. A 200 µl aliquot was taken from each sample and diluted with 200 ul of buffer. Another 200 µl aliquot was taken and diluted with a 100 mM potassium tris(oxalato)chromate (III), 50 mM NaCl solution. The osmotic strength of the buffer and the chromate solutions were previously checked with a vapor pressure osmometer to insure that they were equal to within 1-2%. The samples were vortically mixed.

The EPR spectra were recorded with a Bruker ER 100D spectrometer. Samples were drawn into EPR capillary tubes and sealed. The EPR spectrum of the buffer sample was recorded first, the chromate solution sample was then prepared, and the EPR spectrum of this sample was then immediately recorded. The peak to peak height of the M_{I}=-1 resonance line was used to measure the relative concentrations of the unaffected spin probe in both samples. The total sample volume was proportional to the EPR signal size of buffer diluted aliquot divided by the spectrometer gain setting (ST), while the internal volume of the liposomes was proportional to the EPR signal of the aliquot diluted with chromate solution divided by the gain setting (SI). The internal sample volume (Vi) was given by the SI/ST times the sample volume V. The internal volume, Vi, of the sample was divided by the phosphate content of the sample to give the internal volume per µmole Pi which is the standard way of expressing the internal volume of liposomes.

### EXAMPLE 14

### Encapsulation Efficiency of DPPC LUVET - IF Vesicles as a Function of Initial Size of Liposomes

DPPC (422 mg powder) was hydrated with 21 ml of Tris/NaCl with a trace amount of ¹⁴C sucrose according to the methods of Example 12, for a total concentration of DPPC of 20 mg/ml. Vortical mixing of the sample at 50-55°C resulted in DPPC MLVs. FAT MLVs were prepared according to the methods of Cullis et al., U.S. Patent No. 4,975,282, issued December 4, 1990, for a total of 10 freeze and thaw cycles. The resulting DPPC FATMLVs were extruded ten times using the LUVET apparatus at 60°-65°C according to the methods of Cullis et al. PCT Application PCT/US85/01161, Publication Number WO 86/00238 entitled "Extrusion Techniques for Producing Liposomes", published January 16, 1986, and employing a single 1.0 um Nuclepore polycarbonate filter. Two ml of the LUVET processed liposomes were set aside.

The above method was repeated employing 0.4, 0.2, and 0.1 µm polycarbonate filters, until 2.0 ml LUVET samples of the following diameters were produced: 0.1, 0.2, 0.4, and 1.0 µm.

When FATMLVs were employed for the purposes of this Example, they were used as is, without extrusion. SUVs were prepared according to the methods of Example 12 by sonication of the remaining 0.1 µm filtered LUVETs.

Internal volumes of these liposomes were calculated by ¹⁴C sucrose encapsulation as well as CAT 1 EPR and TEMPONE EPR methods as described hereinabove.

As shown in Figure 6, except for FATMLVs, the internal volume of the IF liposomes increased with decreased size of "starting" liposomes e.g., liposomes prior to the addition of ethanol; these results indicating that the diameter of the starting liposome was an important parameter in determining the final volume of the IF liposome.

### EXAMPLE 15

### Incorporation of DPPG into DPPC IF liposomes

IF liposomes were prepared from DPPC/dipalmitoylphosphatidylglycerol (DPPG) SUVs according to the methods of Example 12 with the following modifications. A total of 30mM phospholipid (DPPC and DPPG) was employed, 0.17 mole fraction (17 mole percent) DPPG. DPPC and DPPG each in chloroform were added to a round bottom flask (50 ml capacity) and mixed well. The lipids were dried to a thin film in the flask by negative pressure (rotary evaporation) and the resulting film hydrated with 2.0 ml Tris/NaCl buffer, and heated to a temperature of 50-55°C. A total concentration of 3.0 M ethanol was employed. A trace amount of ¹⁴C sucrose was added to the sample, and the suspension sonicated to clarity. Upon removal of the ethanol and incubation of the mixture to 50°-55°C, IF liposomes were formed.

The internal volume of the liposomes was calculated by ¹⁴C sucrose encapsulation and TEMPONE EPR methods according to the methods of Example 13.

The above methods were repeated with 1.0, 0.83, 0.66, 0.50 and 0.00 mole fraction (100, 83, 66, 50, and 0 mole percent) DPPG. The results are graphically tabulated in Figure 7A and B.

At higher DPPG fractions, IF liposomes were recovered only in low percentages as the liposomes did not pellet well during the wash step, a problem typical of negatively charged liposomes. The internal volumes of the IF liposomes recovered in the pellets are shown in Figure 7A. The open circles are the volumes measured by ¹⁴C encapsulation, while the closed circles are the volumes measured by the broadening agent (TEMPONE) EPR technique. Figure 7B shows the percent recovery of Pi (closed circles) and ¹⁴C labelled sucrose (open circles) as a function of DPPG.

### EXAMPLE 16

### Captured Volume and Encapsulation as a Function of DPPC Initial Concentration

The materials and procedures of Example 12 were followed to form 2.00 ml of DPPC IF liposomes at 20 mg/ml.

The above methods were repeated employing 2.0, 10.0, 20.0, 80.0, and 160.0 mg of DPPC resulting in five samples of 2.00 ml of DPPC IF liposomes at the following DPPC concentrations: 2.5, 5.0, 10.0, 40.0, and 80.0 mg/ml DPPC.

The ¹⁴C sucrose encapsulation percentage and internal volumes of each of the samples were calculated according to the methods of Example 13.

The results are represented graphically in Figure 8A and B. Figure 8 A demonstrated that the encapsulation of sucrose increases with the initial DPPC lipid concentration. Figure 8 B shows the internal volume of the DPPC IF liposomes measured by both the ¹⁴C sucrose method (open squares) or the EPR method (closed diamonds). The internal volume of the IF liposomes was about 15 to 20 µl/µmole Pi when the initial concentration of DPPC was 1 to 20 mg/ml. Measurement made with the Malvern particle sizer indicated that the average diameter of these liposomes (containing 10 mg/ml and 20 mg/ml lipid) is about 7.0-7.5 µm (see Figure 9A and B). This demonstrates that the internal volume of DPPC liposomes formed by the IF method was much larger than conventional MLVs.

### EXAMPLE 17

### Effect of Cholesterol on Formation of DPPC IF Liposomes

The materials and procedures of Example 12 were followed to make IF liposomes employing DPPC and cholesterol, in a total of 30 mM lipid, using a total concentration of 3.0 M ethanol. The cholesterol and DPPC, provided in stock chloroform solutions at concentrations of 20 mg/ml, were admixed in a 50 ml capacity round bottom flask at 95% DPPC and 5% cholesterol. The lipids were dried to a thin film on the surface of the flask, and hydrated with Tris/NaCl as before. The incubation temperature was 50°-55°C.

Aliquots were removed to assay for cholesterol before and after the IF process, to insure the liposomes contained cholesterol, and to assay for the encapsulation of ¹⁴C sucrose.

The above methods were repeated employing 0.00, 0.02, 0.10, 0.15 and 30.0 mole fraction (0, 2.0, 10, 15 and 30 mole percent) of cholesterol.

The internal volumes of the liposomes were determined by ¹⁴C sucrose encapsulation and both the CAT 1 EPR and TEMPONE EPR methods. The cholesterol content of the IF liposomes was measured using o-phthalaldehyde according to the methods of Rudel and Morris, 1973, J. Lipid Res., 14:14.

Results are graphically represented in Figure 10 A and B. Figure 10A show the "final" cholesterol concentration of the IF liposomes (open circles) and the final percentage of ¹⁴C sucrose entrapped (open squares). As cholesterol content is increased, the amount of encapsulated sucrose decreased.

Figure 10 B shows the decrease in internal volume of the DPPC-cholesterol liposomes as a function of cholesterol content. While the data from the Cat 1 EPR and TEMPONE EPR methods (open triangles and closed circles respectively are in close agreement, the data from the ¹⁴C-sucrose assay shows internal volumes significantly higher. Without being bound to theory, the ¹⁴C-sucrose appears to be "sticking" to the liposomes thus giving readings for higher internal volume.

These studies and Figure 10 indicate that the size of the IF liposomes decreases sharply with increasing cholesterol content, a conclusion that was supported by results of Malvern particle sizing; IF liposomes containing 0% cholesterol had an average diameter of 7.66 µm while 30% cholesterol IF liposomes were 3.99 µm.

### EXAMPLE 18

### Effect of Dioleoylphosphatidylcholine (DOPC) on Formation of DPPC IF Liposomes

The materials and procedures of Example 12 were followed to make IF liposomes employing DPPC and dioleoylphosphatidylcholine ("DOPC"), an unsaturated lipid, in a total of 30 mM lipid, using a total concentration of 3.0 M ethanol. The initial liposomes were formed of DPPC and DOPC, provided in stock chloroform solutions at concentrations of 20 mg/ml, which were admixed in a round bottom flask, with 0.20 mole fraction (20 mole percent) DOPC. The lipids were dried to a thin film on the surface of the flask via rotary evaporation, and hydrated with Tris/NaCl as before. The incubation temperature was 50°-55°C.

The above methods were repeated employing 0.00, 0.11, 0.55, 0.72 and 1.00 mole friction (0, 11, 55, 72, and 100 mole percent) of DOPC.

The internal volume of the IF liposomes was measured by the ¹⁴C sucrose encapsulation method and the TEMPONE EPR method, and the results are demonstrated graphically in Figure 11 A.

As can be seen from the graph, increasing the amount of DOPC decreased the size of the IF liposomes. Even is little as 10 % DOPC appeared to reduce the liposome volume by over 50%. Above 0.4 mole fraction (40 mole percent) DOPC the ethanol-lipid gel did not form and the SUVs did not appear to fuse. The internal volumes of the liposomes at 0.6 and 0.8 mole fraction DOPC were 0.2 and 0.24 µl/µmole Pi respectively which is in the SUV range. In addition, the percent of lipids recoverable by centrifugation decreased above 0.4 mole fraction DOPC (see Figure 11 B).

### EXAMPLE 19

### Entrapment of Radiocontrast Agent Ioversol in DSPC IF Liposomes

DSPC (200 mg, lyophilized powder) was suspended in 5.0 ml distilled water and sonicated to a transluscent SUV suspension (time of sonication was about 20 minutes). The SUV suspension was centrifuged for 10 minutes at 10,000 x g to pellet the titanium residue; SUVs were decanted from the titanium pellet. Ioversol (Optiray 320^{R}, Mallinckrodt) (11.5 mg), 4.1 ml ethanol, and 1.7 ml distilled water were admixed and 3.3 ml aliquots of this mixture were pipetted into three 15 ml capacity Corex^{R} tubes. Aliquots (1.1 ml) of the SUV suspension were added to each tube. The tubes were capped and vortexed vigorously, resulting in a transluscent gel.

The tubes were allowed to set at room temperature for one hour, then uncapped and incubated at 70°C in an immersion bath for one hour with intermittent vortical mixing. Following the incubation, the tubes were N₂ sparged by bubbling a gentle stream of N₂ through the mixture for about 8 minutes. After cooling the contents to room temperature, buffer (30 mM Tris, 150 mM NaCl, 0.6 mM Na₂EDTA, pH 6.7) was added to each tube and mixed by inversion. The unentrapped ioversol was removed by centrifugation washes (3 minutes at 5,000 x g) which were repeated 3 times.

The resulting ioversol entrapped in the IF liposomes was assayed spectrophotometrically by absorption at 245 nm and regression against a standard curve of ioversol in ethanol. Lipid concentration was determined by the method of Chen et al. The entrapment results are shown below in Table 6.

**Table 6**

| Sample | mg/ml iodine | mg/ml DSPC | Final Iodine:Lipid |
|---|---|---|---|
| 1 | 92.1 +/- 7.1 | 14.3 +/- 0.2 | 6.4 (range 5.9-7.0) |
| 2 | 93.2 +/- 8.1 | 13.7 +/- 0.7 | 6.8 (range 5.9-7.8) |
| 3 | 93.2 +/- 9.2 | 13.7 +/- 0.1 | 6.8 (range 6.1-7.5) |

The IF liposomes had a mean diameter of 4.0-5.0 µm determined by Malvern particle size analysis.

### EXAMPLE 20

### Entrapment of Radiocontrast Agent Ioxoglate in DSPC IF Liposomes

The materials and procedures of Example 19 were followed thereby entrapping the radiocontrast agent ioxoglite (Hexibrix^{R}, Mallinckrodt) in IF liposomes. Entrapment was assayed according to the methods of Example 19 and the results tabulated in Table 7 below.

**Table 7**

| Contrast Agent | mg/ml Iodine | mg/ml Lipid | Iodine:Lipid |
|---|---|---|---|
| Ioxoglate | 64.3 +/- 2.7 | 11.5 +/- 0.3 | 5.2-6.0 |

### EXAMPLE 21

### Entrapment of Radiocontrast Agent Iopamidol in DSPC IF Liposomes

The materials and procedures of Example 19 were followed thereby entrapping the radiocontrast agent iopamidol (Isovue^{R}, Bristol-Myers Squibb) in IF liposomes. Entrapment was assayed according to the methods of Example 19 and the results tabulated in Table 8 below.

**Table 8**

| Contrast Agent | mg/ml Iodine | mg/ml Lipid | Iodine:Lipid |
|---|---|---|---|
| Iopamidol | 52.3 +/- 5.2 | 12.0 +/- 0.6 | 3.7-5.0 |

### EXAMPLE 22

### Effect of Incubation Time on Internal Volume of IF Liposomes

The materials and procedures of Example 12 were followed, using 20 mg/ml DPPC and wherein the ethanol concentration was 3.0 M, wherein the incubation period of the gel at a temperature above and below the Tm was varied. The incubation period was set at 5 minutes, and the internal volume of the resulting IF liposomes was measured by the ¹⁴C sucrose encapsulation method (solid bar), and both the CAT 1 EPR (shaded bar), and TEMPONE EPR method (diagonal bar). The results of the measurements are demonstrated on the histogram of Figure 12.

The above methods were repeated wherein the incubation periods were 30 minutes, one hour, and two hours. Similarly, internal volume measurements were made and compared.

As shown by Figure 12, there appears to be no significant difference in the IF liposome internal volume as a function of incubation time between 5 minutes and 2 hours.

### EXAMPLE 23

### Effect of Changing Incubation Procedure on the Internal Volume of IF Liposomes

The materials and procedures of Example 12 were repeated wherein the incubation conditions were varied, e.g., wherein the DPPC SUVs were incubated at room temperature only, without incubation above the DPPC Tm (e.g., at 50-55°C). The internal volume of the resulting IF liposomes was determined by the ¹⁴C sucrose encapsulation method (solid bars) as well as both the CAT 1 EPR (shaded bars), and TEMPONE EPR methods (diagonal bars , and the results are shown on the histogram as "RT" of Figure 12.

The above procedure was repeated without room temperature incubation, wherein the ethanol was added and the incubation was conducted at a temperature above the DPPC Tm (50-55°C). Since the liposomes resulting from this method of incubation did not pellet, rather than centrifugation washes, the liposomes were washed by diluting the DPPC sample 6 fold with 10 ml of Tris/NaCl buffer, removing a 4.0 ml sample, and concentrating this sample with an Amicon 30K microconcentrator (Grace Co.) which was spun for 1 hour at 30,000 x g in a Beckman J-2 centrifuge. The sample volume which was retained was concentrated again using the same procedure. The results of this sample are labelled "50°C".

By reference to Figure 12, it is apparent that both incubation periods are required for the formation of large IF liposomes.

### EXAMPLE 24

### Comparison of Internal Volume of IF Liposomes to MLVs

### I

DPPC (80 mg, powdered form) (Avanti Polar Lipids) was added to 2.00 ml of Tris/NaCl buffer according to the methods of Example 12. The DPPC suspension was sonicated to clarity as described in Example 12. A final concentration of 3.0 M ethanol (addition of 0.43 ml ethanol) was employed thereby forming IF liposomes. The sample was incubated and washed by centrifugation as in Example 12, and the internal volume was determined twice by the CAT 1 EPR methods described in Example 13, the results tabulated in Table 9 hereinbelow.

### II

The above methods or section I were repeated employing 80 mg DPPC in 2.00 ml Tris/NaCl buffer, vortexing at 50-55°C thereby producing MLVs. However, these MLVs were not sonicated and no ethanol was added.

The identical methods were repeated wherein 40 mg of DPPC was employed. The sample was incubated and washed by centrifugation as in Example 12, and the internal volume was determined twice by the CAT 1 EPR methods described in Example 13, the results tabulated in Table 9 hereinbelow.

### III

The methods of section II were repeated wherein 0.43 ml of ethanol for a final concentration of 3.0 M ethanol in the final solution was added. The sample was incubated and washed by centrifugation as in Example 12, and the internal volume was determined twice by the CAT 1 EPR methods described in Example 13, the results tabulated in Table 9 hereinbelow.

Table 9 below illustrates the relatively large internal volume of IF liposomes compared with conventional MLVs or MLVs exposed to relatively high ethanol concentrations.

**Table 9**

| Sample | Lipid Concentration (mg/ml) | 3.0 M EtOH | Internal Volume (µL/µmole Pi) |
|---|---|---|---|
| DPPC SUVs | 20 | yes | 15.78 |
| DPPC SUVs | 40 | yes | 13.38 |
| DPPC MLVs | 20 | yes | 2.02 |
| DPPC MLVs | 40 | yes | 1.70 |
| DPPC MLVs | 20 | no | 1.37 |
| DPPC MLVs | 40 | no | 0.92 |

### EXAMPLE 25

### Iotrolan-DSPC IF Liposomes

The materials and procedures of Example 7 were followed, thus producing an IF liposome population containing iotrolan.

### EXAMPLE 26

### Entrapment of Radiocontrast Agent Iopromide in DSPC IF Liposomes

The materials and procedures of Example 21 are followed thus producing IF liposomes containing iopromide.

## Claims (Claims for the following Contracting State(s): AT, BE, CH, LI, DE, DK, FR, GB, IT, LU, NL, SE)

1. A method of making an interdigitation-fusion gel comprising interdigitating fusing sized liposomes in the presence of an amount of an inducer effective to fuse said liposomes and wherein the sized liposomes have a diameter of less than 0,4 µm.

2. The method of claim 1, wherein the sized liposomes have a diameter of less than 0.05 µm.

3. The method of claim 2, wherein the sized liposomes have a diameter of less than 0,025 µm.

4. The method of claim 1, wherein the sized liposomes comprise a liposome-forming amount of a saturated phospholipid.

5. The method of claim 4, wherein the saturated phospholipid is a symmetrical saturated phospholipid.

6. The method of claim 5, wherein the symmetrical saturated phospholipid is selected from dimyristoyl phosphatidylcholine, distearoyl phosphatidylcholine, dipalmitoyl phosphatidylcholine, dimyristoyl phosphatidylserine, dipalmitoyl phosphatidylserine, distearoyl phosphatidylserine, dimyristoyl phosphatidylethanolamine, dipalmitoyl p hosphatidylethanolamine, distearoyl phosphatidylethanolamine, dimyristoyl phosphatidic acid, distearoyl phosphatidic acid, dipalmitoyl phosphatidic acid, dimyristoyl phosphatidylinositol, distearoyl phosphatidylinositol, dipalmitoyl phosphatidylinositol, hydrogenated soy phosphatidylcholine, dipaimitoyl phosphatidylglycerol, di-0-hexadecyl phosphatidylcholine, distearoyl phosphatidylglycerol and dimyristoyl phosphatidylglycerol.

7. The method of claim 4, wherein the liposomes comprise an additional lipid selected from saturated phospholipids, unsaturated phospholipids, glycolipids, glycosphingolipids, cholesterol and water soluble derivatives thereof and alpha-tocopherols.

8. The method of claim 1, wherein the liposomes comprise a self-inducing lipid.

9. The method of claim 1, wherein the inducer is a bioactive agent.

10. The method of claim 9, wherein the bioactive agent is selected from radiocontrast agents, NMR contrast agents, antimicrobial agents and peptides.

11. The method of claim 10, wherein the bioactive agent is a radiocontrast agent selected from iohexol, iopamidol, ioxaglate, iotrolan, ioversol, iothalamate, iodipamide, iopromide, metrizamide, ioopentol, iodixanol and diatrizoate.

12. The method of claim 11, wherein the radiocontrast agent is iotrolan.

13. The method of claim 1, wherein the inducer is selected from short-chain alchohols, polyols, anaesthetics, buffers and chaotropic salts.

14. The method of claim 13, wherein the inducer is a short-chain alcohol selected from methanol, ethanol, propanol and n-butanol.

15. The method of claim 14, wherein the inducer is ethanol.

16. The method of claim 15, wherein the effective amount of ethanol is an amount equal to 5% to 20 % of the weight of the said composition.

17. The method of claim 16, wherein the effective amount of ethanol is an amount equal to 7% of the weight of the said composition.

18. The method of claim 1, wherein the liposomes comprise a saturated phospholipid and wherein the inducer and the phospholipid are included in a final weight ratio of from 1:10 to 15:1.

19. The method of claim 1, further comprising incubating the sized liposomes in the presence of an additional solute.

20. The method of claim 1, further comprising incubating the interdigitation-fusion gel at a temperature effective to produce an interdigitation-fusion liposome from the gel.

21. The method of claim 20, wherein the interdigitation-fusion gel comprises a saturated phospholipid and wherein the incubation temperature is greater than the transition temperature of the saturated phospholipid in the gel.

22. The method of claim 21, comprising removal of the inducer.

23. An interdigitation-fusion liposome comprising a saturated phospholipid and an inducer in an amount effective to fuse said liposomes, wherein the weight ratio of the inducer to the phospholipid is from 1:10 to 15:1.

24. The interdigitation-fusion liposome of claim 23, wherein the inducer is a bioactive agent.

25. The interdigitation-fusion liposome of claim 24, wherein the bioactive agent is a radiocontrast agent selected from iohexol, iopamidol, ioxaglate, iotrolan, ioversol, iothalamate, iodipamide, iopromide, metrizamide, iopentol, iodixanol and diatrizoate.

26. The interdigitation-fusion liposome of claim 25, wherein the radiocontrast agent is iotrolan.

27. The interdigitation-fusion liposome of claim 23, wherein the weight ratio of inducer to phospholipid is greater than 2:1.

## Claims (Claims for the following Contracting State(s): ES, GR)

1. A method of making an interdigitation-fusion gel comprising interdigitating fusing sized liposomes in the presence of an amount of an inducer effective to fuse said liposomes and wherein the sized liposomes have a diameter of less than 0,4 µm.

2. The method of claim 1, wherein the sized liposomes have a diameter of less than 0,05 µm.

3. The method of claim 2, wherein the sized liposomes have a diameter of less than 0,025 µm.

4. The method of claim 1, wherein the sized liposomes comprise a liposome-forming amount of a saturated phospholipid.

5. The method of claim 4, wherein the saturated phospholipid is a symmetrical saturated phospholipid.

6. The method of claim 5, wherein the symmetrical saturated phospholipid is selected from dimyristoyl phosphatidylcholine, distearoyl phosphatidylcholine, dipalmitoyl phosphatidylcholine, dimyristoyl phosphatidylserine, dipalmitoyl phosphatidylserine, distearoyl phosphatidylserine, dimyristoyl phosphatidylethanolamine, dipalmitoyl phosphatidylethanolamine, distearoyl phosphatidylethanolamine, dimyristoyl phosphatidic acid, distearoyl phosphatidic acid, dipalmitoyl phosphatidic acid, dimyristoyl phosphatidylinositol, distearoyl phosphatidylinositol, dipalmitoyl phosphatidylinositol, hydrogenated soy phosphatidylcholine, dipalmitoyl phosphatidylglycerol, di-0-hexadecyl phosphatidylcholine, distearoyl phosphatidylglycerol and dimyristoyl phosphatidylglycerol.

7. The method of claim 4, wherein the liposomes comprise an additional lipid selected from saturated phospholipids, unsaturated phospholipids, glycolipids, glycosphingolipids, cholesterol and water soluble derivatives thereof and alpha-tocopherols.

8. The method of claim 1, wherein the liposomes comprise a self-inducing lipid.

9. The method of claim 1, wherein the inducer is a bioactive agent.

10. The method of claim 9, wherein the bioactive agent is selected from radiocontrast agents, NMR contrast agents, antimicrobial agents and peptides.

11. The method of claim 10, wherein the bioactive agent is a radiocontrast agent selected from iohexol, iopamidol, ioxaglate, iotrolan, ioversol, iothalamate, iodipamide, iopromide, metrizamide, iopentol, iodixanol and diatrizoate.

12. The method of claim 11, wherein the radiocontrast agent is iotrolan.

13. The method of claim 1, wherein the inducer is selected from short-chain alchohols, polyols, anaesthetics, buffers and chaotropic salts.

14. The method of claim 13, wherein the inducer is a short-chain alcohol selected from methanol, ethanol, propanol and n-butanol.

15. The method of claim 14, wherein the inducer is ethanol.

16. The method of claim 15, wherein the effective amount of ethanol is an amount equal to 5% to 20 % of the weight of the said composition.

17. The method of claim 16, wherein the effective amount of ethanol is an amount equal to 7% of the weight of the said composition.

18. The method of claim 1, wherein the liposomes comprise a saturated phospholipid and wherein the inducer and the phospholipid are included in a final weight ratio of from 1:10 to 15:1.

19. The method of claim 1, further comprising incubating the sized liposomes in the presence of an additional solute.

20. The method of claim1, further comprising incubating the interdigitation-fusion gel at a temperature effective to produce an interdigitation-fusion liposome from the gel.

21. The method of claim 20, wherein the interdigitation-fusion gel comprises a saturated phospholipid and wherein the incubation temperature is greater than the transition temperature of the saturated phospholipid in the gel.

22. The method of claim 21, comprising removal of the inducer.

23. A method of making an interdigitation-fusion liposome, said liposome comprising a saturated phospholipid and an inducer in an amount effective to fuse said liposomes, wherein the weight ratio of the inducer to the phospholipid is from 1:10 to 15:1.

24. The method of claim 23, wherein the inducer is a bioactive agent.

25. The method of claim 24, wherein the bioactive agent is a radiocontrast agent selected from iohexol, iopamidol, ioxaglate, iotrolan, ioversol, iothalamate, iodipamide, iopromide, metrizamide, iapentol, iodixanol and diatrizoate.

26. The method of claim 25, wherein the radiocontrast agent is iotrolan.

27. The method of claim 23, wherein the weight ratio of inducer to phospholipid is greater than 2:1.

## Patentansprüche (Patentansprüche für folgende(n) Vertragsstaat(en): AT, BE, CH, LI, DE, DK, FR, GB, IT, LU, NL, SE)

1. Verfahren zur Herstellung eines Interdigitation-Fusions-Gels, wobei Liposomen bestimmter in größe Anwesenheit einer wirksamen Induktormenge zur Verschmelzung der besagten Liposomen interdigitiert und miteinander verschmelzen werden, dadurch gekennzeichnet, daß die Liposomen bestimmter Größe einen Durchmesser von weniger als 0,4 µm haben.

2. Verfahren nach Anspruch 1, dadurch gekennzeichnet, daß die Liposomen bestimmter Größe einen Durchmesser von Weniger als 0,05 µm haben.

3. Verfahren nach Anspruch 2, dadurch gekennzeichnet, daß die Liposomen bestimmter Größe einen Durchmesser von Weniger als 0,025 µm haben.

4. Verfahren nach Anspruch 1, dadurch gekennzeichnet, daß die Liposomen bestimmter Größe eine liposomenbildende Menge eines gesättigten Phospholipids enthalten.

5. Verfahren nach Anspruch 4, dadurch gekennzeichnet, daß das gesättigte Phospholipid ein symmetrisches gesättigtes Phospholipid ist.

6. Verfahren nach Anspruch 5, dadurch gekennzeichnet, daß das symmetrische gesättigte Phospholipid aus folgenden ausgewählt wird: Dimyristoylphosphatidylcholin, Distearoylphosphatidylcholin, Dipalmitoylphosphatidyicholin, Dimyristoylphosphatidylserin, Dipalmitoylphosphatidylserin, Distearoylphosphatidylserin, Dimyristoylphosphatidylethanolamin, Dipalmitoylphosphatidylethanolamin, Distearoylphosphatidylethanolamin, Dimyristoylphosphatidsäure, Dipalmitoylphosphatidsäure, Distearoylphosphatidsäure, Dimyristoylphosphatidylinositol, Dipalmitoylphosphatidylinositol, Distearoylphosphatidylinositol, hydriertes Soja-Phosphatidylcholin, Dipalmitoylphosphatidylglyzerol, Di-0-Hexadecylphosphatidylcholin, Distearoylphosphatidylglyzerol und Dimyristoylphosphatidylglyzerol.

7. Verfahren nach Anspruch 4, dadurch gekennzeichnet, daß die Liposomen ein zusätzliches Lipid enthalten, das eines der folgenden ist: gesättigte Phospholipide, ungesättigte Phospholipide, Glykolipide, Glycosphingolipide, Cholesterin und dessen wasserlösliche Derivate sowie Alpha-Tocopherole.

8. Verfahren nach Anspruch 1, dadurch gekennzeichnet, daß die Liposomen ein selbstinduzierendes Lipid umfassen.

9. Verfahren nach Anspruch 1, dadurch gekennzeichnet, daß der Induktor eine bioaktive Substanz ist.

10. Verfahren nach Anspruch 9, dadurch gekennzeichnet, daß die bioaktive Substanz ein Radiokontrastmittel, ein Kernspinresonanz-Kontrastmittel, eine antimikrobielle Substanz oder ein Peptid ist.

11. Verfahren nach Anspruch 10, dadurch gekennzeichnet, daß die bioaktive Substanz ein Radiokontrastmittel aus der folgenden Gruppe ist: Iohexol, Iopamidol, Ioxaglat, Iotrolan, Ioversol, Iothalamat, Iodipamid, Iopromid, Metrizamid, Iopentol, Iodixanol oder Diatrizoat.

12. Verfahren nach Anspruch 11, dadurch gekennzeichnet, daß das Radiokontrastmittel Iotrolan ist.

13. Verfahren nach Anspruch 1, dadurch gekennzeichnet, daß der Induktor eines der folgenden ist: kurzkettiger Alkohol, Polyol, Anästhetikum, Puffer oder chaotropes Salz.

14. Verfahren nach Anspruch 13, dadurch gekennzeichnet, daß der Induktor ein kurzkettiger Alkohol, z.B. Methanol, Ethanol, Propanol oder n-Butanol ist.

15. Verfahren nach Anspruch 14, dadurch gekennzeichnet, daß der Induktor Ethanol ist.

16. Verfahren nach Anspruch 15, dadurch gekennzeichnet, daß die wirksame Ethanolmenge 5 %-20 % des Gewichts besagter Zusammensetzung entspricht.

17. Verfahren nach Anspruch 16, dadurch gekennzeichnet, daß die wirksame Ethanolmenge 7 % des Gewichts besagter Zusammensetzung entspricht.

18. Verfahren nach Anspruch 1, dadurch gekennzeichnet, daß die Liposomen ein gesättigtes Phospholipid umfassen, wobei der Induktor und das Phospholipid im endgültigen Gewichtsverhältnis von 1:10 bis 15:1 eingeschlossen sind.

19. Verfahren nach Anspruch 1, dadurch gekennzeichnet, daß die Liposomen bestimmter Größe außerdem in Anwesenheit eines zusätzlichen gelösten Stoffes inkubiert werden.

20. Verfahren nach Anspruch 1, dadurch gekennzeichnet, daß das Interdigitation-Fusions-Gel außerdem bei einer wirksamen Temperatur zur Herstellung eines Interdigitation-Fusions-Liposoms aus dem Gel inkubiert wird.

21. Verfahren nach Anspruch 20, dadurch gekennzeichnet, daß das Interdigitation-Fusions-Gel ein gesättigtes Phospholipid umfaßt, wobei die Inkubationstemperatur über der Übergangstemperatur des gesättigten Phospholipids im Gel liegt.

22. Verfahren nach Anspruch 21, dadurch gekennzeichnet, daß der Induktor entfernt wird.

23. Interdigitation-Fusions-Liposom, das ein gesättigtes Phospholipid und einen Induktor in ausreichender Menge enthält, um besagte Liposomen zu verschmelzen, dadurch gekennzeichnet, daß das Gewichtsverhältnis des Induktors zu den Phospholipiden zwischen 1:10 und 15:1 liegt.

24. Interdigitation-Fusions-Liposom gemäß Anspruch 23, dadurch gekennzeichnet, daß der Induktor eine bioaktive Substanz ist.

25. Interdigitation-Fusions-Liposom gemäß Anspruch 24, dadurch gekennzeichnet, daß die bioaktive Substanz ein Radiokontrastmittel aus der folgenden Gruppe ist: Iohexol, Iopamidol, Ioxaglat, Iotrolan, Ioversol, Iothalamat, Iodipamid, Iopromid, Metrizamid, Iopentol, Iodixanol oder Diatrizoat.

26. Interdigitation-Fusions-Liposom gemäß Anspruch 25, dadurch gekennzeichnet, daß das Radiokontrastmittel Iotrolan ist.

27. Interdigitation-Fusions-Liposom gemäß Anspruch 23, dadurch gekennzeichnet, daß das Gewichtsverhältnis von Induktor zu Phospholipid größer als 2:1 ist.

## Patentansprüche (Patentansprüche für folgende(n) Vertragsstaat(en): ES, GR)

1. Verfahren zur Herstellung eines Interdigitation-Fusions-Gels, wobei Liposomen bestimmter in größe Anwesenheit einer wirksamen Induktormenge zur Verschmelzung der besagten Liposomen interdigitiert und miteinander verschmelzen werden, dadurch gekennzeichnet, daß die Liposomen bestimmter Größe einen Durchmesser von weniger als 0,4 µm haben.

2. Verfahren nach Anspruch 1, dadurch gekennzeichnet, daß die Liposomen bestimmter Größe einen Durchmesser von weniger als 0,05 µm haben.

3. Verfahren nach Anspruch 2, dadurch gekennzeichnet, daß die Liposomen bestimmter Größe einen Durchmesser von weniger als 0,025 µm haben.

4. Verfahren nach Anspruch 1, dadurch gekennzeichnet, daß die Liposomen bestimmter Größe eine liposomenbildende Menge eines gesättigten Phospholipids enthalten.

5. Verfahren nach Anspruch 4, dadurch gekennzeichnet, daß das gesättigte Phospholipid ein symmetrisches gesättigtes Phospholipid ist.

6. Verfahren nach Anspruch 5, dadurch gekennzeichnet, daß das symmetrische gesättigte Phospholipid aus folgenden ausgewählt wird: Dimyristoylphosphatidylcholin, Distearoylphosphatidylcholin, Dipalmitoylphosphatidylcholin, Dimyristoylphosphatidylserin, Dipalmitoylphosphatidylserin, Distearoylphosphatidylserin, Dimyristoylphosphatidylethanolamin, Dipalmitoylphosphatidylethanolamin, Distearoylphosphatidylethanolamin, Dimyristoylphosphatidsäure, Dipalmitoylphosphatidsäure, Distearoylphosphatidsäure, Dimyristoylphosphatidylinositol, Dipalmitoylphosphatidylinositol, Distearoylphosphatidylinositol, hydriertes Soja-Phosphatidylcholin, Dipalmitoylphosphatidylglyzerol, Di-0-Hexadecylphosphatidylcholin, Distearoylphosphatidylglyzerol und Dimyristoylphosphatidylglyzerol.

7. Verfahren nach Anspruch 4, dadurch gekennzeichnet, daß die Liposomen ein zusätzliches Lipid enthalten, das eines der folgenden ist: gesättigte Phospholipide, ungesättigte Phospholipide, Glykolipide, Glycosphingolipide, Cholesterin und dessen wasserlösliche Derivate sowie Alpha-Tocopherole.

8. Verfahren nach Anspruch 1, dadurch gekennzeichnet, daß die Liposomen ein selbstinduzierendes Lipid umfassen.

9. Verfahren nach Anspruch 1, dadurch gekennzeichnet, daß der Induktor eine bioaktive Substanz ist.

10. Verfahren nach Anspruch 9, dadurch gekennzeichnet, daß die bioaktive Substanz ein Radiokontrastmittel, ein Kernspinresonanz-Kontrastmittel, eine antimikrobielle Substanz oder ein Peptid ist.

11. Verfahren nach Anspruch 10, dadurch gekennzeichnet, daß die bioaktive Substanz ein Radiokontrastmittel aus der folgenden Gruppe ist: Iohexol, Iopamidol, Ioxaglat, Iotrolan, Ioversol, Iothalamat, Iodipamid, Iopromid, Metrizamid, Iopentol, Iodixanol oder Diatrizoat.

12. Verfahren nach Anspruch 11, dadurch gekennzeichnet, daß das Radiokonrastmittel Iotrolan ist.

13. Verfahren nach Anspruch 1, dadurch gekennzeichnet, daß der Induktor eines der folgenden ist: kurzkettiger Alkohol, Polyol, Anästhetikum, Puffer oder chaotropes Salz.

14. Verfahren nach Anspruch 13, dadurch gekennzeichnet, daß der Induktor ein kurzkettiger Alkohol, z.B. Methanol, Ethanol, Propanol oder n-Butanol ist.

15. Verfahren nach Anspruch 14, dadurch gekennzeichnet, daß der Induktor Ethanol ist.

16. Verfahren nach Anspruch 15, dadurch gekennzeichnet, daß die wirksame Ethanolmenge 5 %-20 % des Gewichts besagter Zusammensetzung entspricht.

17. Verfahren nach Anspruch 16, dadurch gekennzeichnet, daß die wirksame Ethanolmenge 7 % des Gewichts besagter Zusammensetzung entspricht.

18. Verfahren nach Anspruch 1, dadurch gekennzeichnet, daß die Liposomen ein gesättigtes Phospholipid umfassen, wobei der Induktor und das Phospholipid im endgültigen Gewichtsverhältnis von 1:10 bis 15:1 eingeschlossen sind.

19. Verfahren nach Anspruch 1, dadurch gekennzeichnet, daß die Liposomen bestimmter Größe außerdem in Anwesenheit eines zusätzlichen gelösten Stoffes inkubiert werden.

20. Verfahren nach Anspruch 1, dadurch gekennzeichnet, daß das Interdigitation-Fusions-Gel außerdem bei einer wirksamen Temperatur zur Herstellung eines Interdigitation-Fusions-Liposoms aus dem Gel inkubiert wird.

21. Verfahren nach Anspruch 20, dadurch gekennzeichnet, daß das Interdigitation-Fusions-Gel ein gesättigtes Phospholipid umfaßt, wobei die Inkubationstemperatur über der Übergangstemperatur des gesättigten Phospholipids im Gel liegt.

22. Verfahren nach Anspruch 21, dadurch gekennzeichnet, daß der Induktor entfernt wird.

23. Verfahren zur Herstellung eines Interdigitation-Fusions-Liposom mit einem gesättigten Phospholipid und einem Induktor in ausreichender Menge, um besagte Liposomem zu verschmelzen, dadurch gekennzeichnet, daß das Gewichtsverhältnis des Induktors zu den Phospholipiden zwischen 1:10 und 15:1 liegt.

24. Verfahren gemäß Anspruch 23, dadurch gekennzeichnet, daß der Induktor eine bioaktive Substanz ist.

25. Verfahren gemäß Anspruch 24, dadurch gekennzeichnet, daß die bioaktive Substanz ein Radiokontrastmittel aus der folgenden Gruppe ist: Iohexol, Iopamidol, Ioxaglat, Iotrolan, Ioversol, Iothalamat, Iodipamid, Iopromid, Metrizamid, Iopentol, Iodixanol oder Diatrizoat.

26. Verfahren gemäß Anspruch 25, dadurch gekennzeichnet, daß das Radiokontrastmittel Iotrolan ist.

27. Verfahren gemäß Anspruch 23, dadurch gekennzeichnet, daß das Gewichtsverhältnis von Induktor zu Phospholipid größer als 2:1 ist.

## Revendications (Revendications pour l'(les) Etat(s) contractant(s) suivant(s): AT, BE, CH, LI, DE, DK, FR, GB, IT, LU, NL, SE)

1. Une méthode de fabrication d'un gel d'entrecroisement-fusion selon laquelle on fusionne et entrecroise des liposomes calibrés en présence d'une quantité d'un inducteur qui soit efficace pour fusionner lesdits liposomes et où les liposomes calibrés ont un diamètre de moins de 0,4 µm.

2. La méthode de la revendication 1, où les liposomes calibrés ont un diamètre de moins de 0,05 µm.

3. La méthode de la revendication 2, où les liposomes calibrés ont un diamètre de moins de 0,025 µm.

4. La méthode de la revendication 1, où les liposomes calibrés comprennent une quantité d'un phospolipide saturé, cette quantité étant susceptible de former des liposomes.

5. La méthode de la revendication 4 où le phospholipide saturé est un phospholipide saturé symétrique.

6. La méthode de la revendication 5, où le phospholipide saturé symétrique est choisi à méme dimyristoylphosphatidylcholine, distéaroylphosphatidylcholine, dipalmitoylphosphatidylcholine, dimyristoylphosphatidylsérine, dipalmitoylphosphatidylsérine, distéaroylphosphatidylsérine, dimyristoylphosphatidyléthanolamine, dipalmitoylphosphatidyléthanolamine, distéaroylphosphatidyléthanolamine, acide dimyristoylphosphatidique, acide distéaroylphosphatidique, acide dipalmitoylphosphatidique, dimyristoylphosphatidylinositol, distéaroylphosphatidylinositol, dipalmitoylphosphatidylinositol, phosphatidylcholine de soya hydrogénée, dipalmitoylphosphatidylglycérol, di-O-hexadécylphosphatidylcholine, distéaroylphosphatidylglycérol, et dimyristoylphosphatidylglycérol.

7. La méthode de la revendication 4, où les liposomes comprennent un lipide supplémentaire choisi à même phospholipides saturés, phospholipides non saturés, glycolipides, glycosphingolipides, cholestérol et dérivés hydrosolubles de ceux-ci et alpha-tocophérols.

8. La méthode de la revendication 1, où les liposomes comprennent un lipide auto-inducteur.

9. La méthode de la revendication 1, où l'inducteur est un agent bioactif.

10. La méthode de la revendication 9, où l'agent bioactif est choisi à même substances de radiocontraste, substances de contraste RMN, antimicrobiens et peptides.

11. La méthode de la revendication 10, où l'agent bioactif est une substance de radiocontraste choisie à même iohexol, iopamidol, ioxaglate, iotrolan, ioversol, iothalamate, iodipamide, iopromide, métrizamide, iopentol, iodixanol et diatrizoate.

12. La méthode de la revendication 11, où la substance de radiocontraste est l'iotrolan.

13. La méthode de la revendication 1, où l'inducteur est choisi à même alcools à chaîne courte, polyols, anesthésiques, tampons et sels chaotropiques.

14. La méthode de la revendication 13, où l'inducteur est un alcool à chaîne courte choisi à même méthanol, éthanol, propanol et n-butanol.

15. La méthode de la revendication 14, où l'inducteur est l'éthanol.

16. La méthode de la revendication 15, où la quantité efficace d'éthanol est une quantité égale à 5 % à 20 % du poids de ladite composition.

17. La méthode de la revendication 16, où la quantité efficace d'éthanol est une quantité égale à 7 % du poids de ladite composition.

18. La méthode de la revendication 1, où les liposomes comprennent un phospholipide saturé et où l'inducteur et le phospholipide sont inclus dans un rapport de poids définitif de 1:10 à 15:1.

19. La méthode de la revendication 1, comprenant ultérieurement l'incubation des liposomes calibrés en présence d'un soluté supplémentaire.

20. La méthode de la revendication 1, comprenant ultérieurement l'incubation du gel d'entrecroisement-fusion à une température qui soit efficace pour produire un liposome d'entrecroisement-fusion à partir du gel.

21. La méthode de la revendication 20, où le gel d'entrecroisement-fusion comprend un phospholipide saturé et où la température d'incubation est supérieure à la température de transition du phospholipide saturé dans le gel.

22. La méthode de la revendication 21, comprenant le retrait de l'inducteur.

23. Un liposome d'entrecroisement-fusion comprenant un phospholipide saturé et un inducteur dans une quantité efficace pour fusionner lesdits liposomes, où le rapport de poids de l'inducteur au phospholipide varie entre 1:10 et 15:1.

24. Le liposome d'entrecroisement-fusion de la revendication 23, où l'inducteur est un agent bioactif.

25. Le liposome d'entrecroisement-fusion de la revendication 24, où l'agent bioactif est une substance de radiocontraste choisie à même iohexol, iopamidol, ioxaglate, iotrolan, ioversol, iothalamate, iodipamide, iopromide, métrizamide, iopentol, iodixanol et diatrizoate.

26. Le liposome d'entrecroisement-fusion de la revendication 25, où la substance de radiocontraste est l'iotrolan.

27. Le liposome d'entrecroisement-fusion de la revendication 23, où le rapport de poids de l'inducteur au phospholipide est supérieur à 2:1.

## Revendications (Revendications pour l'(les) Etat(s) contractant(s) suivant(s): ES, GR)

1. Une méthode de fabrication d'un gel d'entrecroisement-fusion selon laquelle on fusionne et entrecroise des liposomes calibrés en présence d'une quantité d'un inducteur qui soit efficace pour fusionner lesdits liposomes ci où les liposomes calibrés ont un diamètre de moins de 0,4 µm.

2. La méthode de la revendication 1, où les liposomes calibrés ont un diamètre de moins de 0,05 µm.

3. La méthode de la revendication 2, où les liposomes calibrés ont un diamètre de moins de 0,025 µm.

4. La méthode de la revendication 1, où les liposomes calibrés comprennent une quantité d'un phospolipide saturé, cette quantité étant susceptible de former des liposomes.

5. La méthode de la revendication 4 où le phospholipide saturé est un phospholipide saturé symétrique.

6. La méthode de la revendication 5, où le phospholipide saturé symétrique est choisi à même dimyristoylphosphatidylcholine, distéaroylphosphatidylcholine, dipatmitoylphosphatidycholine, dimyristoylphosphatidylsérine, dipalmitoylphosphatidylsérine, distéaroylphosphatidylsérine, dimyristoylphosphatidyléthanolamine, dipalmitoylphosphatidyléthanolamine, distéaroylphosphatidyléthanolamine, acide dimyristoylphosphatidique, acide distéaroylphosphatidique, acide dipalmitoylphosphatidique, dimyristoylphosphatidylinositol, distéaroylphosphatidylinositol, dipalmitoylphosphatidylinositol, phosphatidylcholine de soya hydrogénée, dipalmitoylphosphatidylglycérol, di-O-hexadécylphosphatidylcholine, distéaroylphosphatidylglycérol, et dimyristoylphosphatidylglycérol.

7. La méthode de la revendication 4, où les liposomes comprennent un lipide supplémentaire choisi à même phospholipides saturés, phospholipides non saturés, glycolipides, glycosphingolipides, cholestérol et dérivés hydrosolubles de ceux-ci et alpha-tocophérols.

8. La méthode de la revendication 1, où les liposomes comprennent un lipide auto-inducteur.

9. La méthode de la revendication 1, où l'inducteur est un agent bioactif.

10. La méthode de la revendication 9, où l'agent bioactif est choisi à même substances de radiocontraste, substances de contraste RMN, antimicrobiens et peptides.

11. La méthode de la revendication 10, où l'agent bioactif est une substance de radiocontraste choisie à même iohexol, iopamidol, ioxaglate, iotrolan, ioversol, iothalamate, iodipamide, iopromide, métrizamide, iopentol, iodixanol et diatrizoate.

12. La méthode de la revendication 11, où la substance de radiocontraste est l'iotrolan.

13. La méthode de la revendication 1, où l'inducteur est choisi à même alcools à chaîne courte, polyols, anesthésiques, tampons et sels chaotropiques.

14. La méthode de la revendication 13, où l'inducteur est un alcool à chaîne courte choisi à même méthanol, éthanol, propanol et n-butanol.

15. La méthode de la revendication 14, où l'inducteur est l'éthanol.

16. La méthode de la revendication 15, où la quantité efficace d'éthanol est une quantité égale à 5 % à 20 % du poids de ladite composition.

17. La méthode de la revendication 16, où la quantité efficace d'éthanol est une quantité égale à 7 % du poids de ladite composition.

18. La méthode de la revendication 1, où les liposomes comprennent un phospholipide saturé et où l'inducteur et le phospholipide sont inclus dans un rapport de poids définitif de 1:10 à 15:1.

19. La méthode de la revendication 1, comprenant ultérieurement l'incubation des liposomes calibrés en présence d'un soluté supplémentaire.

20. La méthode de la revendication 1, comprenant ultérieurement l'incubation du gel d'entrecroisement-fusion à une température qui soit efficace pour produire un liposome d'entrecroisement-fusion à partir du gel.

21. La méthode de la revendication 20, où le gel d'entrecroisement-fusion comprend un phospholipide saturé et où la température d'incubation est supérieure à la température de transition du phospholipide saturé dans le gel.

22. La méthode de la revendication 21, comprenant le retrait de l'inducteur.

23. Méthode de fabrication d'un liposome d'entrecroisement-fusion comprenant un phospholipide saturé et un inducteur dans une quantité efficace pour fusionner lesdits liposomes, où le rapport de poids de l'inducteur au phospholipide varie entre 1:10 et 15:1.

24. Methode de la revendication 23, où l'inducteur est un agent bioactif.

25. Méthode de la revendication 24, où l'agent bioactif est une substance de radiocontraste choisie à même iohexol, iopamidol, oxaglate, iotrolan, ioversol, iothalamate, iodipamide, iopromide, métrizamide, iopentol, iodixanol et diatrizoate.

26. Méthode de la revendication 25, où la substance de radiocontraste est l'iotrolan.

27. Méthode de la revendication 23, où le rapport de poids de l'inducteur au phospholipide est supérieur à 2:1.
